# EUROPEAN PATENT SPECIFICATION

(11) **EP 3 942 301 B1**
(45) Date of publication and mention of the grant of the patent: **06.05.2026**
(21) Application number: 20719333.5
(22) Date of filing: 23.03.2020
(51) Int. Cl.: G01N 33/68, G01N 33/50

(54) **CATSPER-ACTIVITY-TEST FOR DETERMINING MALE INFERTILITY**
CATSPER-AKTIVITÄTSTEST ZUR BESTIMMUNG DER MÄNNLICHEN UNFRUCHTBARKEIT
TEST D'ACTIVITÉ CATSPER POUR DÉTERMINER L'INFERTILITÉ MASCULINE

(30) Priority: 22.03.2019 EP 19164512; 25.03.2019 LU 101160
(43) Date of publication of application: 26.01.2022
(73) Proprietor: Truion GmbH, 48149 Münster (DE)
(72) Inventor: SCHIFFER, Christian, 41516 Grevenbroich (DE); BRENKER, Christoph, 48159 Münster (DE); STRÜNKER, Timo, 49143 Münster (DE); YOUNG, Samuel Zachary, 48149 Münster (DE)
(74) Representative: Schiweck Weinzierl Koch Patentanwälte Partnerschaft mbB
(86) International application number: PCT/EP2020/057904
(87) International publication number: WO 2020/193446

(56) References cited:
- EP-B1- 2 567 236
- US-A1- 2005 142 566
- US-A1- 2014 212 891
- MOLECULAR PROBES: "MAN0001834 | MP03008 Calcium Calibration Buffer Kits Catalog numbers C3008MP, F6774", 20 February 2014 (2014-02-20), XP055589451, Retrieved from the Internet <URL:https://assets.thermofisher.com/TFS-Assets/LSG/manuals/CalciumCalibrationBufferKits_PI.pdf> [retrieved on 20190516]
- INNOPROT: "INNOVATIVE TECHNOLOGIES IN BIOLOGICAL SYSTEMS CELL-BASED ASSAY KITS COLORIMETRIC CALCIUM ASSAY KIT", 11 April 2011 (2011-04-11), XP055589510, Retrieved from the Internet <URL:http://www.innoprot.com/documentos/archivos/productos/201141112252_en__Ca%20Colorimetric.pdf> [retrieved on 20190516]
- POLINA V LISHKO ET AL: "CatSper: a unique calcium channel of the sperm flagellum", CURRENT OPINION IN PHYSIOLOGY, vol. 2, 1 April 2018 (2018-04-01), pages 109 - 113, XP055589102, ISSN: 2468-8673, DOI: 10.1016/j.cophys.2018.02.004
- REN D ET AL: "A sperm ion channel required for sperm motility and male fertility", NATURE, MACMILLAN JOURNALS LTD, LONDON, vol. 413, no. 6856, 11 October 2001 (2001-10-11), pages 603 - 609, XP002253117, ISSN: 0028-0836, DOI: 10.1038/35098027
- CARLSON A E ET AL: "CatSper1 required for evoked Ca2+ entry and control of flagellar function in sperm", PROCEEDINGS OF THE NATIONAL ACADEMY OF SCIENCES OF THE UNITED STATES OF AMERICA, NATIONAL ACADEMY OF SCIENCES, US, vol. 100, no. 25, 9 December 2003 (2003-12-09), pages 14864 - 14868, XP002977879, ISSN: 0027-8424, DOI: 10.1073/PNAS.2536658100
- XIANG-HONG SUN ET AL: "The Catsper channel and its roles in male fertility: a systematic review", REPRODUCTIVE BIOLOGY AND ENDOCRINOLOGY, vol. 15, no. 1, 15 August 2017 (2017-08-15), XP055589122, DOI: 10.1186/s12958-017-0281-2

## Description

### FIELD OF THE INVENTION

The present invention relates to an *in vitro* method for determining or diagnosing infertility of a male subject comprising the steps of: (a) contacting a sperm sample obtained from said subject with a calcium-free test solution, (b) detecting the motility of the sperm in said sample under the test condition of step (a), and (c) determining infertility of the male subject based on the motility detected in step (b). Further, the present invention relates to a kit for use in a method for determining or diagnosing infertility of a male subject comprising (a) a calcium-free test solution, and (b) a calcium-containing control solution, further comprising a validation solution comprising a CatSper-inhibitor, and further preferably comprising instructions for use and/or an imprint. Furthermore the present invention relates to a use of a kit comprising (a) a calcium-free test solution, and (b) a calcium-containing control solution in an in vitro method for determining or diagnosing infertility of a male subject.

### BACKGROUND OF THE INVENTION

Unintentional childlessness is a serious problem that is caused by male infertility in approximately 40-50% of the cases. However, both the infertility per se and the underlying causes are often unreliable to diagnose. In about 30% of male patients they remain unclear ("idiopathic infertility" or "unexplained sperm dysfunction"). This is due to the lack of suitable and timely diagnostic methods.

In order to access and fertilize the ovum at the end of the fallopian tube, human spermatozoa have a complex behavioral repertoire. The oocyte and surrounding cells secrete e.g. hormones to which the sperm react. It is believed that sperm may adjust their swim direction along the hormone gradient and track the ovum via chemotaxis (Ralt et al., 1991, Proc. Natl. Acad. Sci. USA, 88; Giojalas et al., 1998, Int. J. Androl., 21; Eisenbach et al, 2006, Nat Rev Mol Cell Biol 7(4):276-285; Publicover et al. 2008, Front Biosci 13: 5623 - 5637). The egg cell is surrounded by a protective egg shell (zona pellucida) and a dense cloud of cumulus cells - mechanical barriers for the sperm. In order to penetrate the cumulus cell cloud, the sperm thus change to so-called "hyperactive" swimming behavior: The normal, symmetrical flagellar strike, with which they cover long distances, changes to an asymmetric, low-frequency pattern, which applies the necessary mechanical force to penetrate the cumulus cell cloud (Suarez et al., 1991, Biol Reprod, vol. 44; Ren et al., 2001, Nature 413, 603-609; Quill et al., 2003, Proc Natl Acad Sci USA, vol. 100). The key to overcoming the zona pellucida is, in addition to hyperactivation, acrosomal exocytosis: hydrolytic enzymes stored in the acrosome are released by contact with the zona pellucida (Tulsiani et al., 1998, Experimental Cell Research 240; Gupta et al., 2011, American Journal of Reproductive Immunology 2011*)* and digest the zona pellucida glycoproteins. Only after the zona pellucida has been overcome, the sperm can fuse with the egg and fertilize it (Wassarman et al., 2001, Nat. Cell Biol. 3*).*

Hyperactive swimming, acrosomal exocytosis, and capacitation, an indispensable process of maturation in the female genital tract, through which sperm become fertile, are controlled by intracellular Ca²⁺ (Publicover et al., 2007, Nat Cell Biol, vol. 9*);* it is also believed that the successful navigation of sperm through the fallopian tube also involves [Ca²⁺]ᵢ-changes (Breitbart, 2002, Mol. Cell Endocrinol. 187: 139-144*;* Publicover et al., 2008, Front Biosci 13*).* The Ca^{2 +}-influx into the sperm from outside is crucial for triggering such changes in Ca²⁺. In human sperm, the Ca²⁺ influx is controlled by the sperm-specific CatSper (cation channel of sperm) Ca²⁺ channel that is located in the cell membrane of the sperm flagellum. The CatSper channel is essential for sperm function and fertilization. Mice lacking CatSper are infertile. CatSper-deficient sperm are unable to overcome the egg shell or the cumulus cell cloud because they can not hyperactivate (Ren et al., 2001, Nature 413*;* Carlson et al., 2003, Proc Natl Acad Sci USA, 100*)* and fail to reach the ovum. However, CatSper is expendable for sperm production and its basal motility.

Hence, despite the indispensability of the CatSper function for fertilization success, no diagnostic procedure in the repertoire of andrology laboratories is currently able to detect the absence or a functional defect of the CatSper channel. Although sperm functionality is already determined based on microscopically easily detectable parameters like the amount of sperm and sperm showing progressive motility or morphological alterations, only easily detectable alterations of sperm motility can be diagnosed this way. Further, until now it has been considered safe to study the activity of CatSper in spermatozoa only with labor-intensive and cost-intensive fluorescence optical or electrophysiological measurements, which could not be integrated into the routine diagnostic process of andrology.

The US patent application publication US 2005/0142566 A1 discloses a method of diagnosing a CatSper2-related disorder by determining the presence or absence of a mutation in a CatSper2 gene or protein. The US patent application published as US 2014/0212891 A1 is directed to a method of determining male infertility by measuring the level of a NOX5 protein in a sperm. The European patent EP 2 567 236 B1 is directed to a method of determining male fertility by by determining the expression of CYP24A1 in a semen sample.

However, this contrasts with the high utility of diagnostic information about the CatSper status of a child-desired patient. In fact, in the absence or upon severe functional defects of the CatSper channel, three of the four standard techniques of reproductive medicine - cycle optimization (CO; in German: VZO), intrauterine insemination (IUI), and the classic *in vitro* feritilisation (IVF) - are doomed to failure. Sperm which have no active CatSper channels can only successfully fertilize in the context of intracytoplasmic sperm injection (ICSI), which, however - as the most cost-intensive method - is often only at the end of a fertility treatment history.

If the cause of male infertility remains unexplained after all currently available diagnostic methods have been exhausted, no evidence-based treatment decision for fertility treatment can be made. If evidence-based decisions are substituted by a "trial and error" principle, the treatment means a game of chance for the concerned couple.

Thus, there is a need in the art to close the diagnostic gap in idiopathic male infertility, thereby providing a safe, reliable and easy to handle method. In sum, there is a demand in the art to provide new means and methods that help to diagnose or detect infertility of a male subject which is not characterized by reduced sperm numbers, altered morphology, and/or severly atypic swimming behavior, and which allows for an evidence-based assisted reproductive therapy. The technical problem underlying the present application is thus to comply with these needs. The technical problem is solved by providing the embodiments reflected in the claims, described in the description and illustrated in the examples and figures that follow.

### SUMMARY OF THE INVENTION

The scope of the invention is defined in the appended claims. Embodiments and descriptions not falling under the scope of the appended claims are considered merely as examples suitable for understanding the invention. The present inventors have developed a novel, fundamentally simpler assay compared to fluorescence optical or eletrophysiological methods to screen sperm for functional CatSper channels, which can be used for a reliable detection or diagnosis of infertility of a male subject. Thereby, the determination of the CatSper channel activity in sperm obtained from a male subject allows the attending physician of for example a couple desiring a child an evidence-based therapy decision. In fact, if missing or severly disturbed CatSper channel activity is revealed despite the otherwise inconspicuous spermiogram, this is an exclusion criterion for three out of four of the common reproductive medical treatments strategies (CO, IUI, IVF).

In particular, the present inventors have found an *in vitro* test assay, which is safe, reliable and easy to handle, which determines or diagnoses infertility of a male subject by detecting sperm motility in a calcium-free environment. The simplicity of the assay of the present invention is demonstrated in that it can be performed directly with the native ejaculate, thus eliminating the need for a preceding labour-intensive sperm purification. Further, no specific devices are necessary to use in order to determine or diagnose a male subject with infertility according to the present invention. Finally, the amount of work for the analysis of the motility of the sperm itself is very limited. Thus, by applying the assay of the present invention, the outcome or results whether the male subject is indeed determined or diagnosed with infertility are available very quickly.

In sum, the present invention deals with detecting sperm motility in a calcium-free environment to determine CatSper channel activity and diagnosing male infertility.

Accordingly, in a first aspect, the present invention relates to an *in vitro* method for determining or diagnosing infertility of a male subject comprising the steps of:
a) contacting a sperm sample obtained from said subject with a calcium-free test solution,
b) detecting the motility of the sperm in said sample under the test condition of step (a), and
c) determining infertility of the male subject based on the motility detected in step (b).

The present invention may also comprise the method as described elsewhere herein, wherein no significant change in the amount of motile sperm under the test condition of step (b) is indicative for infertility of said subject.

Further being envisaged herein is the method as described elsewhere herein, wherein a calcium-free test solution comprises less than about 20 nM free calcium and/or wherein the calcium-free test solution comprises at least one agent reducing the free calcium concentration, wherein the at least one agent reducing the free calcium concentration is preferably a chemical binding Ca²⁺. Preferably, the chemical binding Ca²⁺ being the at least one agent reducing the free calcium concentration is any one of EGTA, EDTA, BAPTA, HEDTA, NTA, DiBrBAPTA, citric acid, phosphate, or a Ca²⁺-indicator dye, more preferably EGTA.

Also comprised by the present invention may be the method as described elsewhere herin, wherein step (a) further comprises applying at least one activation stimulus of the CatSper channel, wherein the activation stimulus is preferably applied prior to, simultaneously or after applying the calcium-free test solution to the sperm sample.

Also envisaged herein is the method as described elsewhere herein, wherein the CatSper activation stimulus is any substance and/or condition increasing the CatSper channel activity. Preferably, the substance increasing the CatSper channel activity is a ligand. More preferably, the ligand is any one of a steroid, prostaglandin, a synthetic or a natural chemical that mimics the action of a steroid or prostaglandin, a serum albumin, a zona pellucida protein, or a combination thereof. Most preferably, the steroid is progesterone. The condition increasing the CatSper channel activity preferably comprises increasing the intracellular pH and/or depolarizing the membrane potential.

The present invention may also envisage the method as defined elsewhere herein, wherein the sperm sample is contacted for at least about 5 minutes with the test solution of step (a).

The present invention may also encompass the method as described elsewhere herein, wherein motile sperm is characterized by any movement in comparison to motionless sperm.

It may also be comprised herein the method as described elsewhere herein, wherein the motility of the sperm is determined using a light microscope, and/or wherein determining the amount of motile sperm comprises counting at least about 10 sperm under the test condition of step (b).

Additionally, in a preferred embodiment of the method as described elsewhere herein, the method may further comprise comparing the motility of the sperm detected in step (b) to the motility of the sperm from said subject detected under a control condition comprising a calcium-containing solution. Preferably, the comparison of the motility detected under the test condition and the control condition is indicative for infertility of said subject.

Also comprised by the present invention and as described elsewhere herein for the particular embodiment of the present invention is that the comparison of the motility of the sperm under the test condition and the control condition comprises determining a CatSper activity index, comprising the steps of
a) obtaining a percentage value of motile sperm under i) the test condition and ii) the control condition;
b) subtracting the percentage value of motile sperm under the test condition of i) from the percentage value of motile sperm under the control condition of ii), thereby obtaining a subtraction score;
c) determining the ratio of the subtraction score to the percentage value of motile sperm under the control condition of ii); and
d) multiplying the ratio of step c) with the number 100, thereby obtaining the CatSper-Activity-Index.

The present invention may also envisage for this particular preferred embodiment further comprising comparing the index with a predetermined reference value. Preferably, if the index is below the reference value, it is indicative for infertility of said subject. The reference value being obtained by the particular embodiment of the present invention may be about 20%.

The present invention may also comprise the method as described elsewhere herein, wherein the male subject is a vertebrate. Preferably, the vertebrate being used as male subject in the method of the present invention is a mammal. More preferably, the mammal is any one of a human, a cattle, a buffalo, a horse, a donkey, a camel, a sheep, a goat, a pig, a dog, a cat, a rat, or a mouse, preferably a human.

Further, according to another aspect, the present invention refers to a kit for use in a method for determining or diagnosing infertility of a male subject comprising (a) a calcium-free test solution, and (b) a calcium-containing control solution, further comprising a validation solution comprising a CatSper-inhibitor, and further preferably comprising instructions for use and/or an imprint.

The present invention may also encompass the kit for use as described elsewhere herein, wherein the calcium-free test solution comprises at least one agent reducing the free calcium concentration.

Preferably, the at least one agent reducing the free calcium concentration, which is comprised in the calcium-free test solution, is a chemical binding Ca²⁺. More preferably, the chemical binding Ca²⁺ is any one of EGTA, EDTA, BAPTA, HEDTA, NTA, DiBrBAPTA, citric acid, phosphate, or a Ca²⁺-indicator dye, most preferably EGTA.

The present invention may also envisage the kit for use as described elsewhere herein, wherein the calcium-free test solution and/or the calcium-containing control solution comprises at least one activation stimulus of the CatSper channel.

Preferably, the activation stimulus being comprised in the calcium-free test solution and/or the calcium-containing control solution is any one of a ligand, a weak base, a buffer comprising increased potassium concentration, a potassium- or chloride ion channel inhibitor, or a sodium ionophore. More preferably, the ligand is any one of a steroid, prostaglandin, a synthetic or a natural chemical that mimics the action of a steroid or prostaglandin, a serum albumin, a zona pellucida protein, or a combination thereof. Most preferably, the steroid is progesterone.

Further, according to another aspect, the present invention refers to a use of a kit comprising (a) a calcium-free test solution, and (b) a calcium-containing control solution in an in vitro method for determining or diagnosing infertility of a male subject.

### BRIEF DESCRIPTION OF THE FIGURES

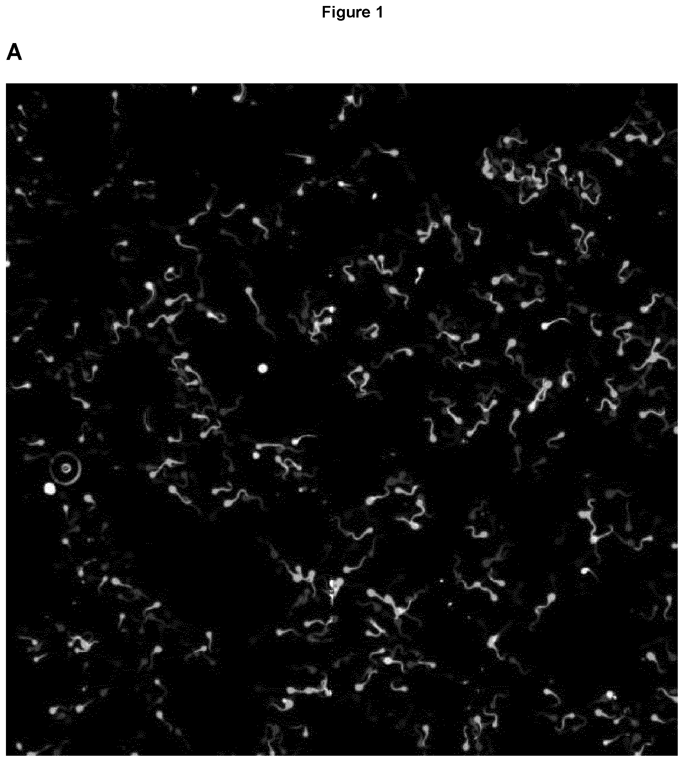

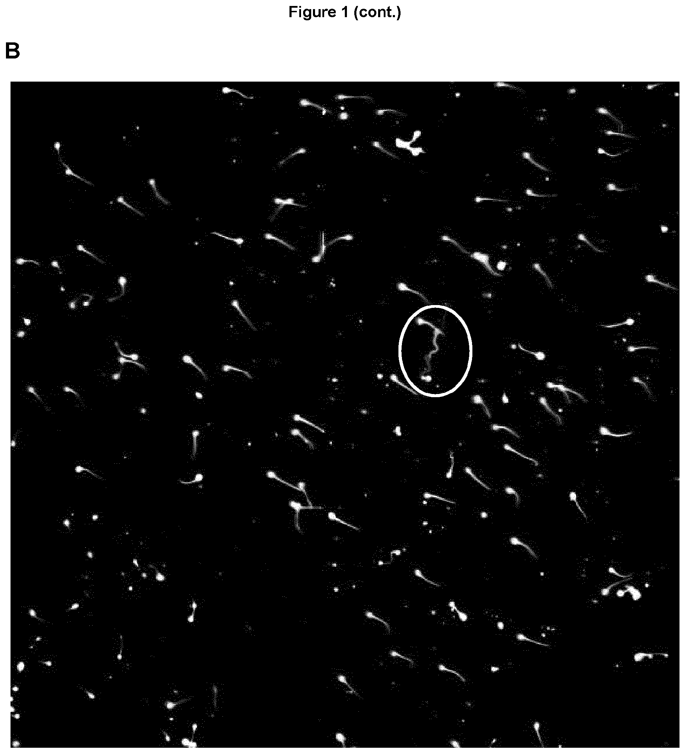

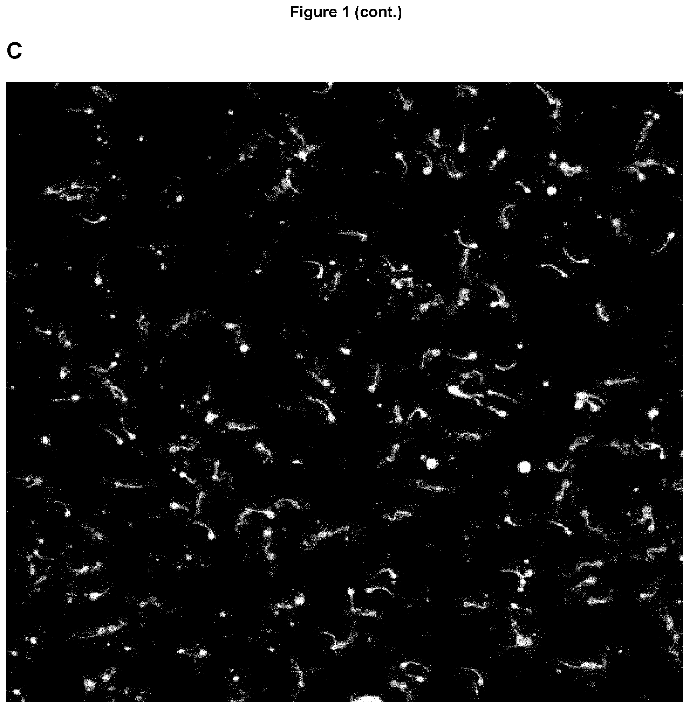

Fig. 1: Sequential frames from video recordings of sperm suspensions after incubation (15 min).
   Control solution (A), Ca²⁺-free test solution in the presence of progesterone (10 µM) depicting immotile sperm from a healthy donor with active CatSper (B), Ca²⁺-free test solution in the presence of progesterone (10 µM) and the CatSper-blocker RU1968F1 (30 µM) depicting motile sperm from a healthy donor with pharmacologically blocked CatSper activity (C). The recording time is grey-scale coded: t= 0 s, light grey; 1 s, medium grey; 2 s, dark grey. Immotile sperm appear white in superposition of the three grey intensities (see (B) with the exception of motile sperm being highlighted by the circle).

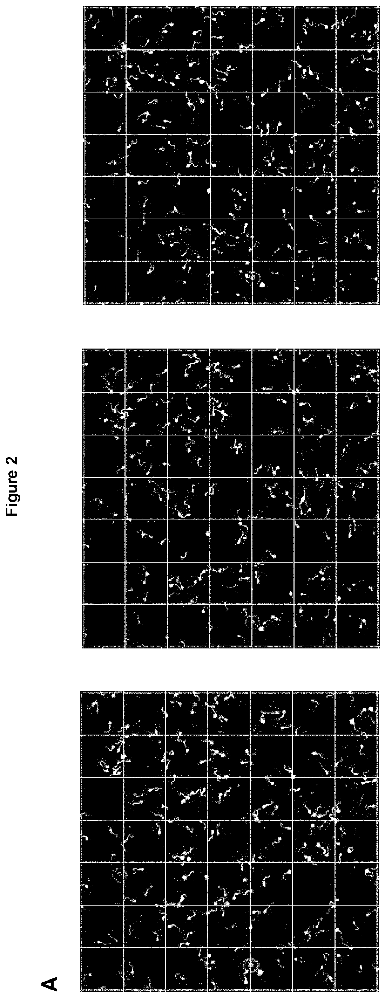

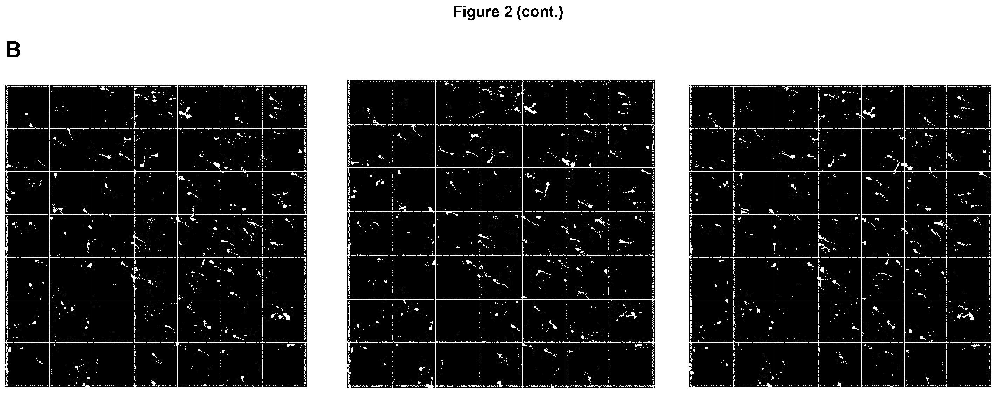

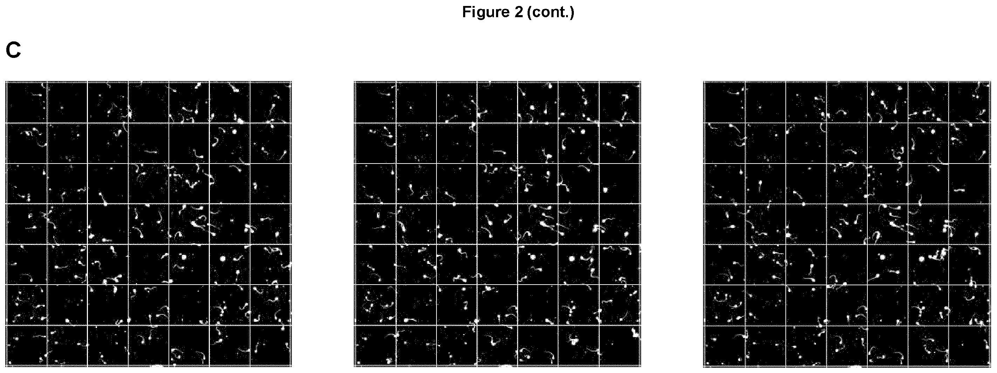

Fig. 2: Consecutive frames from video recordings of the sperm suspensions after incubation (15 min).
   Control solution (A), Ca²⁺-free test solution in the presence of progesterone (10 µM) depicting immotile sperm from a healtyh donor with active CatSper (B), Ca²⁺-free test solution in the presence of progesterone (10 µM) and the CatSper-blocker RU1968F1 (30 µM) depicting motile sperm from a healthy donor with pharmacologically blocked CatSper activity (C). The recording time corresponds to t = 0 s, left; 2 s, in the middle; 4 s, right.

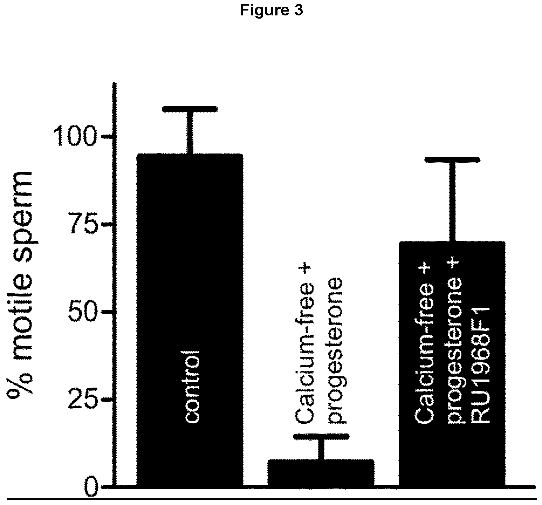

Fig. 3: Sperm motility after incubation in the test solution.
   Relative motility changes in populations of sperm from healthy donors after incubation (15 min) in different test solutions (calcium-containing control solution, calcium-free test solution + progesterone; calcium-free test solution + progesterone + the CatSper-inhibitor RU1968F1) compared to the motility of sperm in the calcium-containing control solution about 30 seconds after starting the incubation (set to 100%). Shown are mean values with standard deviations. Control solution = Ca²⁺, n = 8; Test solution(s) = Ca²⁺-free + progesterone (10 µM), n = 8; Ca²⁺-free + progesterone (10 µM) + the CatSper-inhibitor RU1968F1 (30 µM), n = 6.
Fig. 4: Characterization of CATSPER2-deficient human sperm by the CatSper-Activity-Index.
   CatSper-Activity-Index of n = 467 patients. Framed triangles represent n = 5 patients with loss of CatSper function.
Fig. 5: Verification of CATSPER2-deficient human sperm identified by the CatSper-**Activity-Index using electrophysiological recordings.**
   (A) Representative monovalent CatSper currents in *CATSPER2*^{*-*/*-*} sperm (Patients A-E) and in sperm from a healthy donor (Control), and corresponding current-voltage relationship (right). The membrane voltage was stepped from -100 to +100 mV in increments of 10 mV from a holding potential of -80 mV.
   (B) Outward and inward current amplitudes (mean ± SD) at + 100 mV and -100 mV, respectively, in *CATSPER2*^{*-*/*-*} sperm (Patients A-E) and sperm from healthy donors (Control).

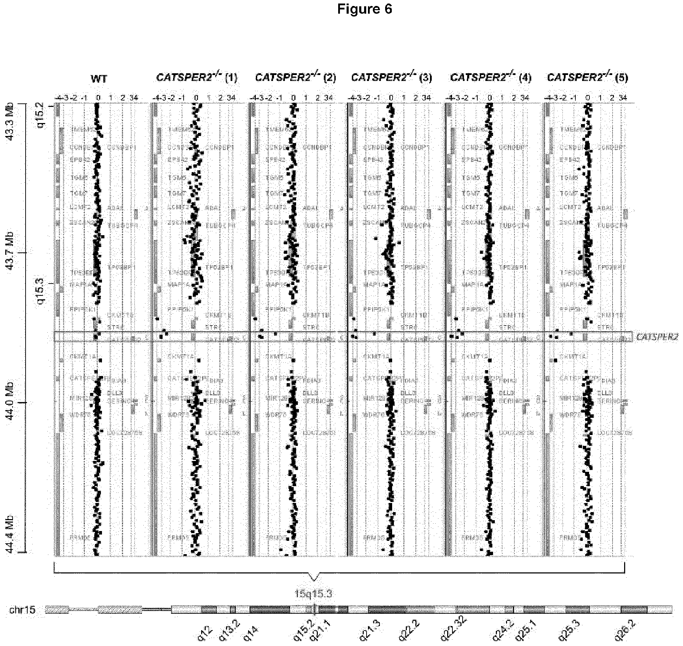

Fig. 6: Array comparative genomic hybridization analysis (array CGH) of CATSPER2-deficient patients identified by the **CatSper-Activity-Index.**
   Array CGH analysis of DNA copy number variants (CNV) in a healthy donor (WT) and the five patients (*CATSPER*^{*-*/*-*} 1-5) identified by the CatSper-Activity-Index. The black squares represent the fluorescence intensity ratios (log2) upon cohybridization of fluorescence-labeled genomic DNA fragments of the healthy donor or patients and controls (reference). Gene dosage variations are indicated by a fluorescence ratio ≠ 0. A ratio of < -1.5 indicates a homozygous deletion.

### DETAILED DESCRIPTION OF THE INVENTION

In order to overcome some of the shortcomings of the means described so far in the prior art for male infertility, the inventors provide herein promising new methods and kits for determing or diagnosing infertility of a male subject based on detecting motility of sperm obtained from said subject under calcium-free test conditions. In particular, the inventors of the present invention surprisingly discovered that male infertility can be reliably detected or diagnosed by the method of the present invention.

In sum, the present invention opens a new avenue for determining or diagnosing infertility of a male subject based on detecting the motility of sperm obtained from said subject under calcium-free test conditions.

As described above, the present invention relates to an *in vitro* method for determining or diagnosing infertility of a male subject comprising the the steps of: (a) contacting a sperm sample obtained from said subject with a calcium-free test solution, (b) detecting the motility of the sperm in said sample under the test condition of step (a), and (c) determining infertility of the male subject based on the motility detected in step (b).

The term *"in vitro"* refers to *ex vivo.*

As used throughout the present invention, the terms "determine" / "determining" or "diagnose" / "diagnosing" include variations like "detect" or "detecting", i.e. these terms may be used interchangeably. According to the present invention, the terms determination / detection / diagnosis are generally understood to refer to the infertility of a male subject. According to the methods and kits of the present invention, by determining, diagnosing or detecting infertility of a male subject using the means described herein, it may be possible to figure out if the subject suffers from infertility.

The term "infertility" being used throughout the present invention refers to the inability of a vertebrate, preferably a mammal, to reproduce by natural means. In the present invention the focus is directed to the infertility of a male subject. In other words, a male subject being infertile may not be able to reproduce himself, hence being able to beget children.

When the method of the present invention is applied, first a sperm sample being obtained from said male subject may be contacted with a calcium-free test solution, which refers to step (a) of the method for determining or diagnosing infertility of a male subject according to the present invention.

In this context and as used throughout the present invention, the term "contacted" / "contacting with" means adding the sperm sample being obtained from said subject to the calcium-free test solution. Preferably, about 50µl of the sperm sample is added to the calcium-free test solution. More preferably, about 50µl of the sperm sample is added to a volume of 450µl calcium-free test solution adding up to a total volume of 500µl. When referring to the preferred embodiment of the present invention, about 50µl of the sperm sample is also added to the calcium-containing control solution. Preferably, about 50µl of the sperm sample is added to a volume of 450µl calcium-containing control solution adding up to a total volume of 500µl. After adding the sperm sample to a tube (or vial) comprising the calcium-free test solution or the calcium-containing control solution, the tube (or vial) comprising the sperm sample and the calcium-free test solution or the tube (or vial) comprising the sperm sample and the calcium-containing control solution may be gently mixed by inverting and/or low-speed vortexing.

Throughout the present invention the term "sperm" or "spermatozoa" refers to the plurality of more than one sperm cell. Thus, more than one sperm cell refers to sperm of the present invention.

In this context, a "sperm sample" refers to a sample comprising sperm. Such sample may be obtained from said male subject by any method known to those skilled in the art, preferably by an ejaculation. A so called ejaculate refers to the orgasmic effusion of a male adolescent or sexually mature male subject comprising sperm. It is preferred that a sperm sample of the present invention obtained from a male subject by ejaculation does not contain about 90% or more, such as about 91% or more, about 92% or more, about 93% or more, about 94% or more, about 95% or more, about 96% or more, about 97% or more, about 98% or more, about 99% or more or even about 100% motionless sperm. Accordingly, sperm samples obtained from a male subject by ejaculation and containing larger amounts of motionless sperm, such as about 90% or more, such as about 91% or more, about 92% or more, about 93% or more, about 94% or more, about 95% or more, about 96% or more, about 97% or more, about 98% or more, about 99% or more or even about 100% motionless sperm are excluded from the method of the present invention. In other words, such particular sperm samples which have a large amount of initially motionless spermatozoa, i.e. before the actual start of the infertility test, cannot be and are not used for the method of the present invention. Accordingly, the sperm sample being contacted with the calcium-free test solution in step (a) of the method as described elsewhere herein comprises at least about 10% or more, such as about 15% or more, about 20% or more, about 30% or more, about 40% or more, about 50% or more, about 60% or more, about 70% or more, about 80% or more, about 90% or more or about 95 % or more motile sperm. The initial sperm motility can be determined using a calcium-containing control solution or native ejaculate and means for detecting motility of sperm in said control solution before starting the method of the present invention as described elsewhere herein.

As used throughput the present invention, the term "motionless" used in combination with sperm refers to "immotile" sperm, meaning when a sperm cell is called "motionless" or "immotile" it does not move at all. A motile sperm cell (motile sperm) however may be characterized by any movement in comparison to a motionless sperm cell (sperm). Any movement as being used herein means any detectable, residual movement of the flagellum and/or the head of the sperm cell. As known to the skilled person in the art the tail of the sperm cell is also called the flagellum, being the longest part and capable of wave-like motion that propels sperm for swimming and aids in the penetration of the egg. In general, sperm motility depends on the 4 parts of the tail: connecting piece, midpiece, principal piece, and the end piece. The head of a sperm cell contains the nucleus and is surrounded by a thin, flattened sac called acrosome comprising enzymes being used for penetrating the female egg. In sum, any kind of independent / autonomous movement of the sperm cell flagellum and/or the sperm cell head, when the motility of the sperm in the sample being used in the method of the present invention is detected, may be a sufficient requirement for assigning (a) sperm cell (sperm) to the category of (a) motile sperm cell (sperm).

The "calcium-free test solution" according to the methods and kits of the present invention may comprise less than about 20 nM, preferably less than about 15 nM, more preferably less than about 10 nM, more preferably less than about 5 nM, such as less than about 19 nM, less than about 18 nM, less than about 17 nM, less than about 16 nM, less than about 15 nM, less than about 14 nM, less than about 13 nM, less than about 12 nM, less than about 11 nM, less than about 10 nM, less than about 9 nM, less than about 8 nM, less than about 7 nM, less than about 6 nM, less than about 5 nM, less than about 4 nM, less than about 3 nM, less than about 2 nM, or less than about 1 nM free calcium. Accordingly, a solution comprising less than about 20 nM, less than about 15 nM, less than about 10 nM, or less than about 5 nM, such as about 19 nM, about 18 nM, about 17 nM, about 16 nM, about 15 nM, about 14 nM, about 13 nM, about 12 nM, about 11 nM, about 10 nM, about 9 nM, about 8 nM, about 7 nM, about 6 nM, about 5 nM, about 4 nM, about 3 nM, about 2 nM, or about 1 nM free calcium qualifies as calcium-free test solution according to the present invention. The term "test solution" when used according to the methods and kits of the present invention refers to a solution which has a reduced calcium concentration and is used to contact a sperm sample obtained from a male subject to test motility of said sperm under said calcium-free conditions. As mentioned elsewhere herein, said test solution according to the present invention comprises preferably less than about 20 nM of free calcium and is thus called "calcium-free test solution".

As used herein, the term "free calcium" refers to calcium not being bound to proteins such as albumine and/or globuline or not being (complex-)bound to for example bicarbonate, lactate, citrate and/or phosphate. In this context and as used throughout the present invention, the term "calcium" means Ca²⁺.

The "calcium-free test solution" being used in the present invention may comprise at least one agent reducing the free calcium concentration. The term "free calcium reducing agent" may also be used interchangeably with the term "agent reducing the free calcium concentration". Under physiological conditions, the calcium concentration within a sperm cell is about 10.000 lower compared to the extracellular fluid - it is on the order of about 200-500 nM versus about 2 mM. Along this concentration gradient, CatSper may mediate an influx of calcium into the sperm. While calcium pumps or calcium exchangers in the sperm membrane may export calcium ions to the outside, the calcium influx may be compensated and calcium homeostasis may be maintained. By inhibiting calcium influx through CatSper by lowering the free extracellular calcium concentration with at least one agent reducing the free calcium concentration below the intracellular concentration, the intracellular calcium concentration may decrease as Ca²⁺ ions flow out of the cell through the CatSper channel. This decrease in calcium concentration is not tolerated by the sperm, i.e. they become immotile. Thus, with at least one agent being capable of reducing the free calcium concentration, the extracellular calcium concentration may be reduced below the intracellular calcium concentration in the sperm. Therefore, by reducing the free calcium concentration it is meant that the free calcium concentration of a solution is lowered to less than about 20 nM of free calcium as defined above, thereby providing a "calcium-free test solution" described elsewhere herein.

Preferably, the at least one agent reducing the free calcium concentration is a chemical binding Ca²⁺. In this context and as used throughout the present invention, the term "chemical binding Ca²⁺" is any substance or molecule with a distinct molecular composition that is produced by or used in a chemical process and which is able to bind free calcium. More preferably, said chemical binding Ca²⁺ of the present invention is any one of a chelator, citric acid, phosphate or a Ca²⁺-indicator dye, preferably a chelator. Even more preferably, said chemical binding Ca²⁺ of the present invention is any one of ethylene glycol-bis(β-aminoethyl ether)-N,N,N',N'-tetraacetic acid (short: EGTA), ethylenediaminetetraacetic acid (short: EDTA), 1,2-bis(o-aminophenoxy)ethane-N,N,N',N'-tetraacetic acid (short: BAPTA), N-(2-Hydroxyethyl)ethylenediamine-N,N',N'-triacetic acid (short: HEDTA), Nitrilotriacetic acid (short: NTA), dibromo-1,2,-bis(o-aminophenoxy)ethane-N,N,-N',N',tetraacetic acid (short: DiBrBAPTA), citric acid, phosphate, or a Ca²⁺-indicator dye, most preferably EGTA. Said chemical binding Ca²⁺ of the present invention may also be any derivative of EGTA, EDTA, BAPTA, HEDTA, NTA, or DiBrBAPTA.

EGTA, EDTA, BAPTA, HEDTA, NTA, DiBrBAPTA or derivatves thereof being mentioned herein as Ca²⁺-binding agents refer to chelators. The term "chelators" refers to any organic or inorganic compound having two or more ione pairs and, thus, may form more than one coordinate bond with a central (metal)-ion. In fact, said chelators coordinate bivalent metal cations with high affinity and make them unavailable for ion channels. Thus, such chelators may have the ability to fix bivalent or polyvalent cations in stable complexes (so-called chelates). If any one of the chelators as mentioned elsewhere herein may be used as a chemical binding Ca²⁺, at least about 2.5 mM, at least about 3 mM, at least about 3.5 mM, at least about 4 mM, at least about 4.5 mM, at least about 5 mM, at least about 5.5 mM, at least about 6 mM, at least about 6.5 mM, at least about 7 mM, at least about 7.5 mM, at least about 8 mM, at least about 8.5 mM, at least about 9 mM, at least about 9.5 mM, or at least about 10mM, preferably about 2.5 mM, about 3 mM, about 3.5 mM, about 4 mM, about 4.5 mM, about 5 mM, about 5.5 mM, about 6 mM, about 6.5 mM, about 7 mM, about 7.5 mM, about 8 mM, about 8.5 mM, about 9 mM, about 9.5 mM, about 10 mM of said chelator may be used according to the methods and kits of the present invention. More preferably, any number in the range between about 2.5 mM to about 10 mM, between about 2.9 mM to about 8.8 mM, between about 3.3 mM to about 7.5 mM, or between about 4 mM to about 6 mM of said chelator may be used in the calcium-free test solution according to the methods and kits of the present invention. Most preferably, about 5 mM EGTA are used as chemical binding Ca²⁺ in the calcium-free test solution according to the present invention.

A "Ca²⁺-indicator dye" as used herein refers to a reagent indicating the presence, absence or concentration of another substance such as metal ions, here calcium, by means of characteristic color change. Thereby, such Ca²⁺-indicator dye may form a complex with calcium and reduces the free calcium in the test solution. Accordingly, also a Ca²⁺-indicator dye can be used as Ca²⁺-binding agents of the present invention.

According to the method for determining or diagnosing infertility in a male subject described herein, step (a) may further comprise applying at least one activation stimulus of the CatSper channel. The term "CatSper activation stimulus" may be used interchangeably with the term "activation stimulus of the CatSper channel".

As used herein and throughout the present invention, the term "CatSper channel" refers to a spermspecific *cation channel of sperm* Ca²⁺-channel, which is located in the cell membrane of a sperm flagellum. The CatSper channel complex comprises four homologous α subunits (CatSper 1-4) and at least five auxiliary subunits: CatSper β, CatSper γ, CatSper δ, CatSper ε and CatSper 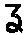 .

The activation stimulus of the CatSper channel may be applied prior to, simultaneously or after applying the calcium-free test solution to the sperm sample in step (a) of the method of the present invention. Thus, when obtaining the sperm sample from the male subject either the activation stimulus may be applied prior to said sperm sample before applying the calcium-free test solution as mentioned in step (a), or both the activation stimulus and the calcium-free test solution may be applied to said sperm sample at the same time in step (a) of the method of the present invention. Also comprised herein is applying the activation stimulus to said sperm sample after having applied the calcium-free test solution as mentioned in step (a) of the method of the present invention. According to the present invention, it is also envisaged that two or more activation stimulus of the CatSper channel described elsewhere herein are used or applied in combination, i.e. simultaneously.

In a preferred embodiment, the CatSper activation stimulus is any substance and/or condition increasing the CatSper channel activity. Thus, it may also be comprised herein that the CatSper activation stimulus is one or more substance(s) increasing the CatSper channel activity. It may also be comprised herein that the CatSper activation stimulus is one or more condition(s) increasing the CatSper channel activity. Also being comprised herein is that the CatSper activation stimulus is a combination of any substance and any condition increasing the CatSper channel activity.

In this context and as used throught the present invention, the term "increasing the CatSper channel activity" means that a larger number of ions are flowing per unit time through the pore of the CatSper channel at a given membrane potential in comparison to the number of ions flowing per unit time through the pore of the CatSper channel at a given membrane potential without being increased by any substance and/or condition as mentioned herein. The term "increase" or "increasing" the CatSper channel activity can also refer to improve / enhance the CatSper channel activity or stimulate / stimulating the CatSper channel activity or relieve / relieving the CatSper channel from inhibition. The term "increase" or "increasing" the CatSper channel activity can also refer to active / activating the CatSper channel activity. When a substance or a condition may stimulate the CatSper channel activity, this means that said substance or condition may stimulate the ion flux through said channel. When a substance or a condition may relieve / release the CatSper channel from inhibition, this means that said substance or condition may help the CatSper channel not being inhibited or blocked anymore.

Preferably, the substance increasing the CatSper channel activity is a ligand. A "ligand" as used herein and throught the present invention refers to a small organic molecule, a protein, a peptide or a chemical that directly binds to said CatSper channel or that binds to a receptor protein that affects the activity of the CatSper channel. Thus, by binding to said channel or receptor protein the "ligand" is able to increase said channel activity. More preferably, the ligand increasing the CatSper channel activity is any one of a steroid, prostaglandin, a synthetic or a natural chemical that mimics the action of a steroid or prostaglandin, a serum albumin, a zona pellucida protein, or a combination thereof.

"Steroids" in general are biologically active organic compounds comprising a core structure of 17 carbon atoms in four rings, arranged in a specific molecular configuration. The preferred steroid according to the present invention is progesterone as a ligand increasing the CatSper channel activity. Especially in human sperm, progesterone and also prostaglandins, which are two compounds of the oviduct that activate human CatSper channels.

The term "synthetic chemical that mimics the action of a steroid or prostaglandin" as used herein refers to any molecule / substance being generated or produced involving synthesis which additionally may imitate what a steroid or prostaglandin might do. A "natural chemical that mimics the action of a steroid or prostaglandin" refers to any molecule / substance being formed by nature which additionally may imitate what a steroid or prostaglandin might do.

Also envisaged according to the methods and kits of the present invention is a combination of two or more of the abovementioned ligands, which may then increase the CatSper channel activity.

The "condition increasing the CatSper channel activity" as mentioned elsewhere herein may comprise increasing the intracellular pH value and/or depolarizing the membrane potential leading to CatSper activation.

In this context, the term "increasing the intracellular pH" refers to increasing the pH inside the sperm cell (intracellular) with a weak base. In other words, increasing the intracellular pH means generating an alkaline pH. A weak base as used throughout the present invention means a base being only partially ionized in aqueous solution and having weak electrolytes. In addition, a base having a pK_{b} value greater than 8, the pK_{b} value indicating to what extent a base is ionized in the equilibrium reaction with water, may be considered as a weak base and is known to the skilled person. A weak base of the present invention may comprise, but is not limited to, ammonium chloride, triethylamin (short: TEA) or imidazole. Any weak base known to the person skilled in the art, which may be used to increase the intracellular pH, may be present in any physiological aqueous solution. Increasing the intracellular pH may be achieved by increasing the extracellular pH (outside the sperm cell). Once the extracellular pH may be alkalized, also the intracellular pH may alkalize (increase).

When the activation stimulus is a condition increasing the intracellular pH and which is applied prior to or after applying the calcium-free test solution to the sperm sample as defined in step (a) of the method for determining or diagnosing infertility of a male subject, the physiological aqueous solution comprising any weak base for increasing the intracellular pH in the sperm cell may not be the same as the calcium-free test solution. In case the activation stimulus is a condition increasing the intracellular pH and which is applied simultaneously with the calcium-free test solution to the sperm sample as defined in step (a) of the method for determining or diagnosing infertility of a male subject, then the calcium-free test solution may comprise any weak base for increasing the intracellular pH.

The term "depolarization" refers to a change within a cell during which the cell undergoes a shift in electric charge distribution, resulting in less negative charge inside the cell. The difference between the electric potential between the interior and the exterior of a cell is called the membrane potential. Such membrane potential can be determined by applying the Goldman-Hodgkin-Katz voltage equation (GHK equation), more commonly known as the Goldman equation as known to the person skilled in the art. Once the membrane potential decreases (i.e. becomes less negative), the membrane potential may be considered as depolarizing.

Thus, as used herein, "depolarizing" refers to shifting the membare potential to less negative or more positive values.

Depolarizing the membrane potential or depolarization of the membrane potential may be achieved by increasing the potassium concentration in the solution being used as activation stimulus to a concentration of higher than about 30 mM. In other words, the term "increasing the potassium concentration" refers to increasing the potassium concentration to a concentration of more than about 30 mM. The following definition referring to "increasing the potassium concentration" in the solution being used as activation stimulus may also be applied to the term "buffer comprising increased potassium concentration" as used according to the present invention.

The causes of the membrane potential are a concentration gradient of the ions over the membrane and their selective permeability. Inside the cell are high concentrations of protein molecules (proteins), which are present as anions at a cell pH of about 7.2; further, in approximately equimolar concentration of positively charged (cations) K + ions (potassium) and in much lower concentrations of Na + and Cl- (sodium and chloride). By contrast, completely different conditions prevail in the extracellular fluid: it contains much less protein molecules and has inverse ratios with respect to the other ions, i. high Na + and CI - ion concentrations and a significantly lower proportion of K +. The membrane potential is determined by the relative permeability (pK, pNa, pCI) of the membrane for the different ions and the concentration difference of the ions across the membrane. The relative permeability of the membrane is usually pK : pNa : pCI = 1 : 0.05 : 0.45. The membrane potential can be calculated by using the Goldman-Hodgkin-Katz equation (GHK equation) as mentioned elsewhere herein. Considering the relative ion permeabilities and the GHK equation, it is clear to a person skilled in the art that an increase in the extracellular K+ concentration depolarizes the membrane potential.

An increased potassium concentration in the solution being used as activation stimulus may increase the CatSper channel activity as described elsewhere herein. When the activation stimulus is a condition depolarizing the membrane potential and which is applied prior to or after applying the calcium-free test solution to the sperm sample as defined in step (a) of the method for determining or diagnosing infertility of a male subject, the solution having an increased potassium concentration for depolarizing the membrane potential may not be the same as the calcium-free test solution. In case the activation stimulus is a condition depolarizing the membrane potential and which is applied simultaneously with the calcium-free test solution to the sperm sample as defined in step (a) of the method for determining or diagnosing infertility of a male subject, then the calcium-free test solution may have an increased potassium concentration for depolarizing the membrane potential.

Further, depolarizing the membrane potential or depolarization of the membrane potential may also be achieved by applying potassium and/or a chloride ion channel inhibitor, or by applying a sodium ionophore. A potassium and chloride ion channel inhibitor reduces pK and pCl, respectively, which, according to the GHK equation, depolarizes the membrane potential. A sodium ionophore according to the present invention depolarizes sperm by increasing pNa.

In this context and as used throughout the present invention, the term "inhibitor" means a modulator which is able to inhibit the activity of something. In particular, the "ion channel inhibitor" depolarizing the membrane potential or depolarization of the membrane potential may either inhibit a potassium or a chloride ion channel, or inhibit both channels.

In this context and as used throughout the present invention, an "ionophore" refers to a chemical molecule being able to reversibly bind ions. An ionophore may be lipid-soluble and transports ions across the cell membrane. An ionophore may also be called ion carrier. An ionophore may also be an antibiotic. An ionophore may be selective for specific cations and anions, thereby exhibiting high affinites for different cations / anions such as Na⁺ or K⁺. According to the present invention an ionophore is thus a synthetical or natural compound capable of increasing the membrane's permeability for (selected) ionic species, such as sodium or potassium. A sodium ionophore of the present invention may comprise, but is not limited to, gramicidin, fusafungin monensin, salinomycin, narasin, preferably gramicidin being selective for potassium and sodium, but impermeable for anions. The ionophor according to the present invention artifically increases the relative permeability of the membrane for Na+ (pNa) and, thereby, depolarizes the membrane potential.

Also envisaged herein, is that the condition increasing the CatSper channel activity comprises a combination of increasing the intracellular pH and depolarizing the membrane potential. Thus, it is also envisaged that the condition increasing the CatSper channel activity comprises a combination of increasing the intracellular pH by applying any weak base such as ammonium chloride and depolarizing the membrane potential by increasing the potassium concentration of the solution being used as at least one activation stimulus. It is also envisaged that the condition increasing the CatSper channel activity comprises a combination of increasing the intracellular pH by applying any weak base such as TEA and depolarizing the membrane potential by increasing the potassium concentration of the solution being used as at least one activation stimulus. It is further envisaged that the condition increasing the CatSper channel activity comprises a combination of increasing the intracellular pH by applying any weak base such as imidazole and depolarizing the membrane potential by increasing the potassium concentration of the solution being used as at least one activation stimulus.

It is also envisaged that the condition increasing the CatSper channel activity comprises a combination of increasing the intracellular pH by applying any weak base such as ammonium chloride and depolarizing the membrane potential by applying potassium and/or a chloride ion channel inhibitor. It is also envisaged that the condition increasing the CatSper channel activity comprises a combination of increasing the intracellular pH by applying any weak base such as TEA and depolarizing the membrane potential by applying a potassium - and/or a chloride ion channel inhibitor. It is further envisaged that the condition increasing the CatSper channel activity comprises a combination of increasing the intracellular pH by applying any weak base such as imidazole and depolarizing the membrane potential by applying potassium and/or a chloride ion channel inhibitor.

It is also envisaged that the condition increasing the CatSper channel activity comprises a combination of increasing the intracellular pH by applying any weak base such as ammonium chloride and depolarizing the membrane potential by applying a sodium ionophore, preferably gramicidin. It is also envisaged that the condition increasing the CatSper channel activity comprises a combination of increasing the intracellular pH by applying any weak base such as TEA and depolarizing the membrane potential by applying a sodium ionophore, preferably gramicidin. It is further envisaged that the condition increasing the CatSper channel activity comprises a combination of increasing the intracellular pH by applying any weak base such as imidazole and depolarizing the membrane potential by applying a sodium ionophore, preferably gramicidin.

The disclosure of the present invention referring to a combination of increasing the intracellular pH by applying any weak base such as ammonium chloride, TEA or imidazole and depolarizing the membrane potential by increasing the potassium concentration of the solution being used as at least one activation stimulus may also be combined with any substance increasing the CatSper channel activity. The disclosure of the present invention referring to a combination of increasing the intracellular pH by applying any weak base such as ammonium chloride, TEA or imidazole and depolarizing the membrane potential by applying a potassium-and/or a chloride ion channel inhibitor may also be combined with any substance increasing the CatSper channel activity. The disclosure of the present invention referring to a combination of increasing the intracellular pH by applying any weak base such as ammonium chloride, TEA or imidazole and depolarizing the membrane potential by applying a sodium ionophore, preferably gramicidin may also be combined with any substance increasing the CatSper channel activity. In particular, the specific disclosure referring to a combination of increasing the intracellular pH by applying any weak base such as ammonium chloride, TEA or imidazole and depolarizing the membrane potential by increasing the potassium concentration of the solution being used as at least one activation stimulus may be combined with any one of the ligands of the present invention mentioned elsewhere herein as substance increasing the CatSper channel activity. In particular, the specific disclosure referring to a combination of increasing the intracellular pH by applying any weak base such as ammonium chloride, TEA or imidazole and depolarizing the membrane potential by applying a potassium- and/or a chloride ion channel inhibitor may be combined with any one of the ligands of the present invention mentioned elsewhere herein as substance increasing the CatSper channel activity. In particular, the specific disclosure referring to a combination of increasing the intracellular pH by applying any weak base such as ammonium chloride, TEA or imidazole and depolarizing the membrane potential by applying a sodium ionophore, preferably gramicidin may be combined with any one of the ligands of the present invention mentioned elsewhere herein as substance increasing the CatSper channel activity. Preferably, the disclosure of the present invention referring to a combination of increasing the intracellular pH by applying any weak base such as ammonium chloride, TEA or imidazole and depolarizing the membrane potential by increasing the potassium concentration of the solution being used as at least one activation stimulus is combined with progesterone as a substance increasing the CatSper channel activity. Preferably, the disclosure of the present invention referring to a combination of increasing the intracellular pH by applying any weak base such as ammonium chloride, TEA or imidazole and depolarizing the membrane potential by applying a potassium- and/or a chloride ion channel inhibitor is combined with progesterone as a substance increasing the CatSper channel activity. Preferably, the disclosure of the present invention referring to a combination of increasing the intracellular pH by applying any weak base such as ammonium chloride, TEA or imidazole and depolarizing the membrane potential by applying a sodium ionophore, preferably gramicidin is combined with progesterone as a substance increasing the CatSper channel activity.

The present invention also envisages that according to the method for determining or diagnosing infertility of a male subject described herein preferably comprising at least one activation stimulus of the CatSper channel applied in step (a) of said method, the sperm sample is contacted for at least about 5 minutes, at least about 6 minutes, at least about 7 minutes, at least about 8 minutes, at least about 9 minutes, at least about 10 minutes, at least about 11 minutes, at least about 12 minutes, at least about 13 minutes, at least about 14 minutes, at least about 15 minutes, at least about 16 minutes, at least about 17 minutes, at least about 18 minutes, at least about 19 minutes, at least about 20 minutes, at least about 30 minutes, at least about 60 minutes or preferably about 5 minutes, about 6 minutes, about 7 minutes, about 8 minutes, about 9 minutes, about 10 minutes, about 11 minutes, about 12 minutes, about 13 minutes, about 14 minutes, about 15 minutes, about 16 minutes, about 17 minutes, about 18 minutes, about 19 minutes, about 20 minutes, about 30 minutes, about 40 minutes, about 50 minutes, about 60 minutes or in the range between about 5 minutes and about 60 minutes, preferably between about 10 minutes and about 25 minutes, preferably between about 12 minutes and about 19 minutes, preferably between about 13 minutes and about 17 minutes with the calcium-free test solution of step (a) before determining infertility of the male subject based on the motility detected in step (b) of the method. In a preferred embodiment of the present invention, the sperm sample is contacted for about 15 minutes with the calcium-free test solution of step (a) before determining infertility of the male subject based on the motility detected in step (b) of the method. In an even more preferred embodiment, the sperm sample is contacted for about 15 minutes with the calcium-free test solution of step (a) at about 37°C before determining infertility of the male subject based on the motility detected in step (b) of the method.

In this specific context, the term "contacted / contacting" also means "incubate" / "incubating". Preferably, "incubate" or "incubating" means incubating by gently mixing such as inverting and/or low-speed vortexing.

The method of the present invention for determining or diagnosing infertility of a male subject necessarily comprise as a second step (b) detecting the motility of the sperm in said sample under the test condition of step (a) and a third step (c) comprising determining infertility of the male subject based on the motility detected in step (b).

In this context, the term "detecting the motility" refers to determining whether the sperm in the sample are motile or immotile as defined elsewhere herein by using any technique for distinguishing between motile and immotile sperm. Thus the term "determing the motility" may also be used interchangeably with the term detecting the motility. Preferably, when detecting / determining whether the sperm in the sample are motile or immotile as defined elsewhere herein, a light microscope is used.

Thus, the motility of the sperm may be determined / detected in step (b) of the method of the present invention by using a light microscope.

The term "light microscope" may also comprise an automated computer assisted sperm movement analysis called CASA, which is known to a person skilled in the art. Therefore, the present invention may also comprise a method of the present invention, wherein the motility of the sperm may be determined / detected in step (b) by using CASA.

When detecting the motility of the sperm in the sperm sample under the test condition of step (a) and there may be no significant change in the amount of motile sperm under the test condition, this may be indicative for infertility of said subject. In other words, the respective subject is considered infertile.

As mentioned elsewhere herein, a calcium flux occurs through said CatSper channel along the concentration gradient of the intra- and extracellular calcium concentration. CatSper-deficient sperm, however, lack the exit port for Ca²⁺ ions once a decrease in the extracellular calcium concentration occurs as mentioned elsewhere herein: their motility is maintained even under calcium-free conditions for extended periods of time. Comparing the ratio of motile versus immotile sperm in a calcium-free test solution which has been contacted with the sperm sample of the present invention may be used to determine whether sperm have functional CatSper channels. If the sperm may remain motile for a long time, there may be probably a CatSper defect or other serious defect in the calcium-dependent signaling process, leading to the conclusion that the male subject may be infertile. If the sperm may be immotile, the CatSper channel is most likely functional.

Thus, the term "no significant change in the amount of motile sperm" means that the amount of motile sperm may not remarkably decrease or that the amount of motile sperm may remain more or less the same when the sperm sample obtained form said male subject is contacted with the calcium-free test solution. In other words, when the term "no significant change in the amount of motile sperm" is used, it means that the amount of motile sperm does not decrease by about 20% or more. It means that less than about 20%, such as about 19% or less, about 18% or less, about 17% or less, about 16%or less, about 15% or less, about 14% or less, about 13% or less, about 12% or less, about 11% or less, about 10% or less, about 9% or less, about 8% or less, about 7% or less, about 6% or less, about 5% or less, about 4% or less, about 3% or less, about 2% or less, about 1% or less, or even 0% of the motile sperm become immotile when the motility of the sperm in said sample as defined elsewhere herein has been detected under the test conditions of step (b) according to the method of the present invention.

Meaning, if the sperm sample may be contacted with the calcium-free test solution as defined in step (a) of the method for determining or diagnosing infertility of a male subject of the present invention and the amount of motile sperm may not decrease or vice versa that the amount of motile sperm may remain similar, the male subject may be considered as being infertile (Fig. **1C** and 2C).

The method for determining or diagnosing infertility of a male subject of the present invention may also comprise that determining the amount of motile sperm, in other words determing / detecting the motility of the sperm may comprise counting of at least about 10, at least about 20, at least about 30, at least about 40, at least about 50, at least about 60, at least about 70, at least about 80, at least about 90, at least about 100, at least about 110, at least about 120, at least about 130, at least about 140, at least about 150, at least about 160, at least about 170, at least about 180, at least about 190, at least about 200, at least about 210, at least about 220, at least about 230, at least about 240, at least about 250, at least about 260, at least about 270, at least about 280, at least about 290, at least about 300, at least about 350, at least about 400, at least about 450, or at least about 500, preferably about 10, about 20, about 30, about 40, about 50, about 60, about 70, about 80, about 90, about 100, about 110, about 120, about 130, about 140, about 150, about 160, about 170, about 180, about 190, about 200, about 210, about 220, about 230, about 240, about 250, about 260, about 270, about 280, about 290, about 300, about 350, about 400, about 450, about 500 or more sperm under the test condition of step (b), for example by using a light microscope. Preferably, determining the amount of motile sperm, in other words determing the motility of the sperm, comprises counting of about 200 sperm under the test condition of step (b), for example by using a light microscope. Thus, when determining the amount of motile sperm for determining or diagnosing infertility of a male subject of the present invention, which comprises counting of at least about 10 sperm under the test condition of step (b) of the method of the present invention, and less than about 20%, such as about 19% or less, about 18% or less, about 17% or less, about 16% or less, about 15% or less, about 14% or less, about 13% or less, about 12% or less, about 11% or less, about 10% or less, about 9% or less, about 8% or less, about 7% or less, about 6% or less, about 5% or less, about 4% or less, about 3% or less, about 2% or less, about 1% or less, or even 0% of the motile sperm become immotile when the motility of the sperm in said sample as defined elsewhere herein has been detected under the test conditions of step (b) according to the method of the present invention, this means that there is no significant change in the amount of motile sperm, therefore being indicative for infertility of said subject.

Alternatively, instead of evaluating single sperm by counting at least 10 sperm under the test condition of step (b), also whole sperm populations may be evaluated, preferably using image fluctuation. To this end the spatiotemporal fluctuations of the intensity in time-lapse images from a sample are analyzed. These fluctuations directly correlate with the mean motility of the sample.

In a preferred embodiment, the method for determining or diagnosing infertility of a male subject of the present invention further comprises comparing the motility of the sperm detected in step (b) to the motility of the sperm from said subject detected under control condition comprising a calcium-containing solution. In this specific embodiment, the sperm sample being obtained from a male subject as defined elsewhere herein may be contacted with a calcium-free test solution as defined in step (a) of the method of the present invention, which is considered as the test condition and the sperm sample may separately be contacted with a calcium-containing solution, which is considered as the control condition. After this step, again the motility of the sperm under the test condition and under the control condition may be detected and compared to each other (Fig. 1A / 2A in comparison to Fig. 1B / 2B or Fig. 1A / 2A in comparison to Fig. 1C / 2C). A relative motility change in sperm populations in different test solutions compared to the motility of the sperm under the control solution is depicted in Fig. 3, which clearly demonstrates that there is no significant change in the amount of motile sperm under the calcium-free test solution for sperm samples comprising a CatSper-inhibitor (which corresponds to sperm samples being CatSper-deficient). Thus, the comparison of the motility detected under the test condition and the control condition may be indicative for infertility of said subject, in other words indicative for the subject being infertile. As mentioned herein, when there is no significant change in the amount of motile sperm under the test condition in comparison to the control condition, the male subject is considered as being infertile.

In this context, the term "calcium-containing solution" refers to a solution comprising more than about 1000 nM, more than about 10.000 nM, more than about 100.000 nM, more than about 1 mM free calcium. The calcium-containing solution may also be called control solution herein. It may correspond to a HTF-buffer (human tubular fluid) as known to the skilled person. The calcium-containing solution of the present invention may comprise an agent reducing the free calcium concentration, but if this is the case such solution comprises more than about 1000 nM, more than about 10.000 nM, more than about 100.000 nM, more than about 1 mM free calcium. In a preferred embodiment of the method of the present invention, the calcium-containing control solution additionally comprises at least one activation stimulus of the CatSper channel as defined elsewhere herein.

Thus, according to a preferred embodiment, the present invention relates to an *in vitro* method for determining or diagnosing infertility of a male subject comprising the steps of a) contacting a sperm sample obtained from said subject with a calcium-free test solution; b) contacting a sperm sample obtained from said subject with a calcium-containing control solution; c) detecting the motility of the sperm under the condition of (a) and (b); and d) comparing the motility of the sperm detected under the test condition of step a) to the motility of the sperm detected under the control condition of step b), wherein the comparison of the motility detected under the test condition and the control condition is indicative for infertility of said subject.

In a further preferred embodiment of the method for determining or diagnosing infertility of a male subject of the present invention, the comparison of the motility of the sperm under the test condition and the control condition comprises determining a CatSper-Activity-Index (CAI).

For determining said particular CatSper-Activity-Index, first a percentage value of motile sperm under the test condition and the control condition may be obtained. In this context, the term "obtaining a percentage value" refes to calculating the proportion / ratio of the amount of motile sperm after having contacted the sperm sample with the test solution as described elsewhere herein and after having contacted the sperm sample with the control solution as described elsewhere herein to the amount of all sperm being counted in the sample under the test condition and under the control condition as described elsewhere herein in the Examples. As used herein, the term "amount of motile sperm" refers to the actual amount / number of sperm having any detectable movement as described elsewhere when being counted using any applicable technique such as a light microscope Thus, the term "percentage value of motile sperm" may be expressed as proportion / ratio of the amount of motile sperm to the amount of all sperm being counted in the sample under the test condition and under the control condition. The amount of motile sperm after having contacted the sperm sample with the test solution (control solution) in relation to all sperm being counted in the sample under the test condition (control condition) may be expressed in terms of a percentage, i.e. the quotient of dividing the amount of motile sperm after having contacted the sperm sample with the test solution (control solution) by all sperm being counted in the sample under the test condition (control condition).

Secondly, for the comparison of the motility of the sperm under the test condition and the control condition comprising determining a CatSper-Activity-Index, the percentage value of motile sperm under the test condition as defined herein may be subtracted from the percentage value of motile sperm under the control condition as described herein in the Examples. In this context, the term "subtract or subtracting" means to calculate the difference between two numbers expressed in %, which define the percentage value as defined above by subtraction. By subtracting the percentage value of motile sperm under the test condition from the percentage value of motile sperm under the control condition, a substration score may be obtained. In this context, the "subtraction score" refers to the result of the subtraction of the percentage value of motile sperm under the test condition from the percentage value of motile sperm under the control condition. Said subtraction score may be expressed in percentage (%) as well.

Thirdly, for the comparison of the motility of the sperm under the test condition and the control condition comprising determining a CatSper-Activity-Index, the ratio of the subtraction score to the percentage value of motile sperm under the control condition may be determined (see the Examples). Particularly, in this specific context, the term "determine or determining" refers to calculate or calculating, e.g. calculating the ratio of the subtraction score to the percentage value of motile sperm under the control condition.

Determining the ratio of the subtraction score to the percentage value of motile sperm under the control condition as defined above means comparing the subtraction score to the percentage value of motile sperm under the control condition, or putting the subtraction score and the percentage value of motile sperm under the control condition into relation to one another. Thus, determining the ratio of the subtraction score in relation to the percentage value of motile sperm under the control condition may also be comprised herein. Thus, the term "in relation to" or just "to" as used herein in this context, e.g. "the level of A" (in relation) to "the level of B", generally means comparing "A" to "B". Preferably, this comparison is carried out by dividing A by B, thereby obtaining a ratio. Here, the relation of "A" to "B" can be expressed as the quotient of "A" divided by "B", again thereby obtaining the ratio.

Fourthly, for the comparison of the motility of the sperm under the test condition and the control condition comprising determining a CatSper-Activity-Index, the ratio as determined above may be multiplied with the number 100. By multiplying the ratio as defined above with the number 100, the CatSper-Activity-Index (short: CAI) may be obtained (see the Examples). The term "multiplying" refers in this context to taking "A" times "B", thus taking the ratio as determined above times the number 100.

Further, the preferred embodiment of the method of the present invention referring to the determination of a CatSper-Activity-Index may additionally comprise the further step of comparing said index determined as described above with a predetermined reference value.

The term "reference value" may be understood to generally refer to a qualitative or quantitative value. Thus, if the reference value may be understood to refer to a qualitative value, said index may be below said reference value. Also comprised herein is, if the reference value may be understood to refer to a quantitative value, said index may deviate from said reference value. In another embodiment, if the reference value may be understood to refer to a quantitative value, said index may deviate from said reference value by about 0.1%, about 0.2% about 0.3%, about 0.4%, about 0.5%, about 0.6%, about 0.7%, about 0.8%, about 0.9%, about 1%, about 1.5%, about 2.0%, about 2.5%, about 3.0%, about 3.5%, about 4.0%, about 4.5%, about 5.0%, about 5.5%, about 6.0%, about 6.5%, about 7.0%, about 7.5%, about 8.0%, about 8.5%, about 9.0%, about 9.5%, about 10%, about 15%, about 20%, about 25%, about 30%, about 35%, about 40%, about 45%, about 50%, about 60%, about 70%, about 80%, about 90%, or about 100%. The greater the deviation of said index from said reference value, the higher the possibility that a subject of the present invention may be infertile.

Selecting a reference value involves, among other things, consideration of the probability of the condition, distribution of true and false determination at different test references, and estimates of the consequences of treatment (or a failure to treat) based on the determination. Suitable references may be determined in a variety of ways. Thus, a "predetermined reference value" may be obtained before hand for example as described below and may then be used as a reference to determine whether a male subject may be infertile / may suffer from infertility.

For example population studies may be used to select a reference value. Receiver Operating Characteristic ("ROC") is often used to select a reference able to best distinguish a well responding subpopulation (a control condition) from a poorly responding subpopulation (a test condition). A false positive in this case occurs when a person tests positive (poor responder being infertile), but actually is a good responder that might be fertile. A false negative, on the other hand, occurs when the person tests negative (good responder might be fertile), when it actually is infertile. To draw a ROC curve, the true positive rate (TPR) and false positive rate (FPR) are determined as the decision reference is varied continuously. Since TPR is equivalent with sensitivity and FPR is equal to 1 - specificity, the ROC graph is sometimes called the sensitivity vs (1 - specificity) plot. A perfect test will have an area under the ROC curve (AUC) of 1.0; a random test will have an area of 0.5. Preferably, the tests described herein provide a ROC curve area greater than 0.5, preferably at least 0.6, more preferably 0.7, still more preferably at least 0.8, even more preferably at least 0.9, and most preferably at least 0.95. A reference value is selected to provide an acceptable level of specificity and sensitivity. A reference that can provide an acceptable level of specificity and sensitivity in separating a population of subjects into "bins" such as a "first" subpopulation (e.g., which is infertile) and a "second" subpopulation, which might be fertile. A reference value is selected to separate this first and second population by one or more of the following measures of test accuracy:
an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;
a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;
a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;
at least about 75% sensitivity, combined with at least about 75% specificity;
a positive likelihood ratio (calculated as sensitivity/(1-specificity)) of greater than 1, at least about 2, more preferably at least about 3, still more preferably at least about 5, and most preferably at least about 10; or
a negative likelihood ratio (calculated as (1-sensitivity)/specificity) of less than 1, less than or equal to about 0.5, more preferably less than or equal to about 0.3, and most preferably less than or equal to about 0.1.

In addition to reference comparisons, other methods for correlating assay results to a patient selection or classification (e.g. likelihood of being a good responder or a poor responder) include decision trees, rule sets, Bayesian methods, and neural network methods. These methods can produce probability values representing the degree to which a subject belongs to one classification out of a plurality of classifications.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1.

The comparison to a reference value may be carried out manually, semi-automatically or in a fully automated manner. In some embodiments, the comparison may be computer assisted. A computer assisted comparison may employ values stored in a database as a reference for comparing an obtained value or a determined amount, for example via a computer implemented algorithm. Likewise, the comparison to a reference measurement may be carried out manually, semi-automatically or in a fully automated manner, including in a computer assisted manner.

According to the present invention, if said index may be below the reference value, it may be indicative for infertility of said subject. The present invention may also comprise that if said index may deviate from a reference value, it may be indicative for infertility of said subject. In a preferred embodiment, if said index may deviate from a reference value by about 0.1%, about 0.2% about 0.3%, about 0.4%, about 0.5%, about 0.6%, about 0.7%, about 0.8%, about 0.9%, about 1%, about 1.5%, about 2.0%, about 2.5%, about 3.0%, about 3.5%, about 4.0%, about 4.5%, about 5.0%, about 5.5%, about 6.0%, about 6.5%, about 7.0%, about 7.5%, about 8.0%, about 8.5%, about 9.0%, about 9.5%, about 10%, about 15%, about 20%, about 25%, about 30%, about 35%, about 40%, about 45%, about 50%, about 60%, about 70%, about 80%, about 90%, or by about 100%, it may be indicative for infertility of said subject. The greater the deviation of said index from said reference value, the higher the possibility that the subject may be infertile / may suffer from infertility.

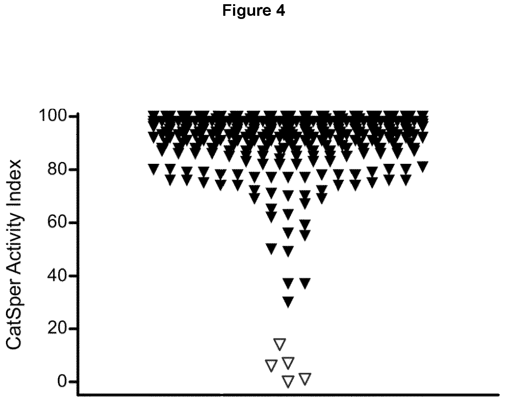

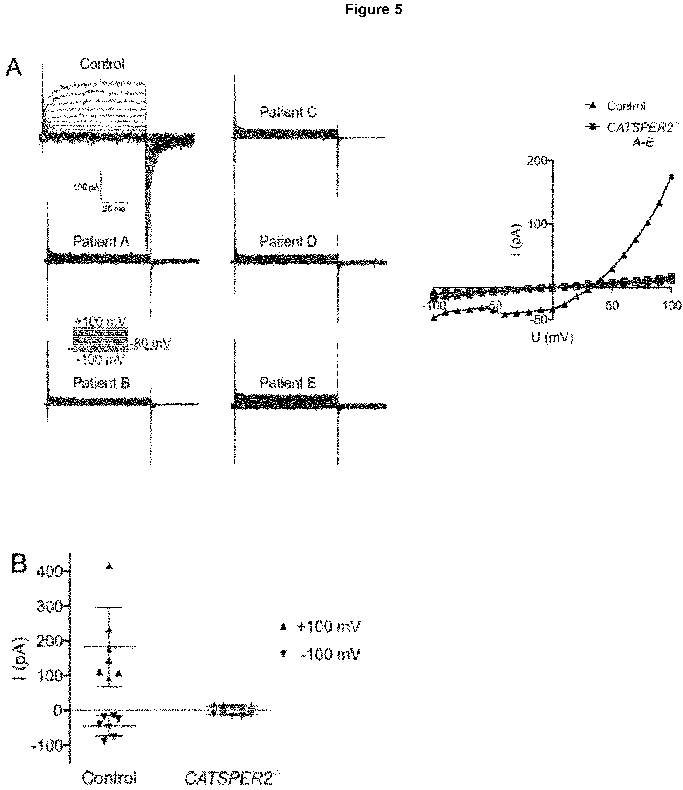

In a preferred embodiment, the reference value is about 20%. Thus, if the CatSper activity index obtained for a male subject whose infertility is under assessment is below the reference value of about 20%, it is indicative that the subject is infertile (Fig. 4). Infertility obtained by the CatSper-Acitivity-Index was verified by electrophysiological recording (Fig. 5) and genetic analysis (Fig. 6).

In this context, the term "below the reference value" means any number, preferably being rounded up to two digits behind the comma, which is below (not equal to) the indicated number of the reference value.

The term "male subject" as used herein and throughout the present invention, also addressed as a male individual, refers to a vertebrate. Preferably, the vertebrate is a mammal. Even more preferably, the mammal is any one of a human, a cattle, a buffalo, a horse, a donkey, a camel, a sheep, a goat, a pig, a dog, a cat, a rat, or a mouse, most preferably a human.

In a further aspect, the present invention provides a kit comprising a calcium-free test solution as defined elsewhere herein and a calcium-containing control solution as defined elsewhere herein. The respective kit is envisaged to be used in the method for determining or diagnosing infertility of a male subject as described elsewhere herein. Accordingly, the present invention also refers to the use of a kit described herein in an *in vitro* method for determining or diagnosing infertility of a male subject as described in great detail elsewhere herein.

According to the present invention, the calcium-containing control solution (short: control solution) and the calcium-free test solution (short: test solution) may each preferably be provided in one container or vial in a kit (pharmaceutical pack). Thus, the present invention may comprise a kit comprising one vial or container comprising the control solution and one vial or container comprising the test solution.

The kit of the present invention may further comprise means for instructions/instructions for use (instruction sheet) and/or an imprint. Such "instructions for use" may refer to a notice in the form prescribed by a governmental agency regulating the manufacture, use and/or sale of such pharmaceuticals or biological products (e.g. control and test solution of the kit of the present invention), reflecting approval by the agency of the manufacture, use and/or sale of the product for human administration or diagnostics. An "imprint" as used herein may illustrate the important information about the products / components of the kit and how they can be used by the skilled person for determining or diagnosing infertility of a male subject. The illustration may be made on a print attached to the kit. Such information may comprise the composition of the components / products of the kit, conditions for storage, the shelf life and/or stability of the products / components of the kit.

The kit of the present invention further comprise a validation solution comprising a CatSper-inhibitor. Any CatSper-inhibitor may be used to inhibit / block the activity of the CatSper channel. Preferably, the kit of the present invention further comprises a validation solution comprising the RU1968F1 CatSper-inhibitor, which is known to a person skilled in the art (Rennhack et al., 2018 British Jorunal of Pharmacology*).* Said specific RU1968F1 inhibitor may pharmacologically mimic the phenotype of a CatSper-deficiency, meaning when applying such validation solution comprising RU1968F1 CatSper-inhibitor, this may correspond to the same effect as if a male subject with absence or functional defect of the CatSper channel being infertile would be used for the method of the present invention. Such CatSper-inhibitor may be used for internal calibration to evaluate / define a reference value of the present invention. Thus, the present invention may comprise a kit comprising one vial or container comprising the control solution, one vial or container comprising the test solution and one vial or container comprising the validation solution comprising a CatSper-inibitor, preferably comprising RU1968F1 CatSper-inhibitor.

Additionally, the kit of the present invention may comprise the calcium-free test solution comprising at least one agent reducing the free calcium concentration as described elsewhere herein. Thus, the present invention may comprise a kit comprising one vial or container comprising the control solution and one vial or container comprising the test solution comprising at least one agent reducing the free calcium concentration as defined elsewhere herein.

Preferably, the at least one agent reducing the free calcium concentration, which may be comprised in the calcium-free test solution of the kit of the present invention, is a chemical binding Ca²⁺ as defined elsewhere herein. More preferably, said chemical binding Ca²⁺ being the at least one agent reducing the free calcium concentration being comprised in the calcium-free test solution of the kit of the present invention is any one of a chelator, citric acid, phosphate or a Ca²⁺-indicator dye, even more preferably a chelator. Most preferably, said chemical binding Ca²⁺ being the at least one agent reducing the free calcium concentration being comprised in the calcium-free test solution of the kit of the present invention is any one of EGTA, EDTA, BAPTA, HEDTA, NTA, DiBrBAPTA, citric acid, phosphate, or a Ca²⁺-indicator dye, most preferably EGTA. Said chemical binding Ca²⁺ being the at least one agent reducing the free calcium concentration being comprised in the calcium-free test solution of the kit of the present invention may also be any derivative of EGTA, EDTA, BAPTA, HEDTA, NTA, or DiBrBAPTA.

The kit of the present invention may further comprise the calcium-free test solution comprising at least one activation stimulus of the CatSper channel as described elsewhere herein. It may also be comprised that the kit of the present invention may comprise the calcium-containing control solution comprising at least one activation stimulus of the CatSper channel. The present invention also envisages a kit, wherein the calcium-free test solution and the calcium-containing control solution comprise at least one activation stimulus of the CatSper channel. Thus, the present invention may also comprise a kit comprising one vial or container comprising the control solution and one vial or container comprising the test solution comprising at least one agent reducing the free calcium concentration as defined elsewhere herein and comprising at least one activation stimulus of the CatSper channel as also defined elsewhere herein. The present invention may further comprise a kit comprising one vial or container comprising the control solution comprising at least one activation stimulus of the CatSper channel as defined elsewhere herein and one vial or container comprising the test solution comprising at least one agent reducing the free calcium concentration as defined elsewhere herein and comprising at least one activation stimulus of the CatSper channel as also defined elsewhere herein. Also comprised herein is a kit comprising one vial or container comprising the control solution, one vial or container comprising the test solution comprising at least one agent reducing the free calcium concentration and further comprising one vial or container comprising a solution comprising at least one activation stimulus of the CatSper channel. Thus, the present invention may also provide a kit comprising a calcium-free test solution and a calcium-containing control solution and further comprising a solution comprising at least one activation stimulus of the CatSper channel.

As defined elsewhere herein, the at least one activation stimulus being comprised in the calcium-free test solution and/or in the calcium-containing control solution of the kit of the present invention or being comprised in a separate solution of the kit of the present invention as defined above may be any one of a ligand, a weak base, a buffer comprising increased potassium concentration, a potassium- or chloride ion channel inhibitor, or a sodium ionophore as described elsewhere herein.

Preferably, the ligand as being used as at least one activation stimulus of the CatSper channel being comprised in the calcium-free test solution and/or in the calcium-containing control solution of the kit of the present invention or being comprised in a separate solution of the kit of the present invention is any one of a steroid, prostaglandin, a synthetic or a natural chemical that mimics the action of a steroid or prostaglandin, a serum albumin, a zona pellucida protein, or a combination thereof, even more preferably the ligand is a steroid, most preferably the steroid is progesterone. When the at least one activation stimulus may be used and which may be separately comprised in a solution of the kit of the present invention other than the calcium-free test solution or the calcium-containing control solution, the kit may further comprise a carrier. In some embodiments, said carrier may also be comprised in one or more containers or vials comprising the solution comprising the at least one activation stimulus as defined elsewhere herein.

The term "carrier" refers to a diluent, adjuvant, or vehicle with which the at least one activation stimulus is compounded / mixed in the solution of the kit comprising the at least one activation stimulus. Such carriers can be sterile liquids, such as water, buffer including PBS, HTF, and oils including those of petroleum, animal, vegetable or synthetic origin, such as peanut oil, soybean oil, mineral oil, sesame oil and the like. The carrier can also be any organic solvent, such as DMSO or an alcohol, preferably ethanol or methanol, or the like as known to the skilled person. Water or HTF is a preferred carrier.

All of the above-mentioned concerning the method of the present invention may be applicable to the aspect of the kit and the use of such kit of the present invention.

It is noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. The term "at least one" refers to one or more such as two, three, four, five, six, seven, eight, nine, ten or more. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "less than" or in turn "more than" does not include the concrete number.

For example, less than 20 means less than the number indicated. Similarly, more than or greater than means more than or greater than the indicated number, e.g. more than 80 % means more than or greater than the indicated number of 80 %.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". When used herein "consisting of" excludes any element, step, or ingredient not specified.

The term "including" means "including but not limited to". "Including" and "including but not limited to" are used interchangeably.

The term "about" means plus or minus 10%, preferably plus or minus 5%, more preferably plur or minus 2%, most preferably plus or minus 1%.

Throughout the description and claims of this specification, the singular encompasses the plural unless the context otherwise requires. In particular, where the indefinite article is used, the specification is to be understood as contemplating plurality as well as singularity, unless the context requires otherwise.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

A better understanding of the present invention and of its advantages will be gained from the following examples, offered for illustrative purposes only. The examples are not intended to limit the scope of the present invention in any way.

### EXAMPLES OF THE INVENTION

### Materials being used in the present invention

The control solution being used in the methods and kits of the present invention corresponds to the HTF buffer (human tubular fluid) used for years in research with sperm (Strünker et al. 2011, Nature 471, 382-386). The calcium-free test solution is also based on this buffer. It is very likely that the test can also be carried out with buffer solutions of a different composition, as long as they are kept in the physiological range.

**Table 1: Composition of the control and the test solutions being used in the methods and the kits of the present invention.**

| | Calcium-containing control-solution | Calcium-free test solution with progesterone |
|---|---|---|
| NaCl [mM] | 93,8 | 93,8 |
| KCl [mM] | 4,7 | 4,7 |
| MgSO₄ [mM] | 0,2 | 0,2 |
| KH₂PO₄ [mM] | 0,37 | 0,37 |
| CaCl₂ [mM] | 2,04 | 0 |
| HEPES [mM] | 21 | 21 |
| Glucose [mM] | 2,78 | 2,78 |
| Na-Lactate [mM] | 21,4 | 21,4 |
| NaHCO₃ [mM] | 4 | 4 |
| EGTA [mM] | 0 | 5 |
| Pyruvate [mM] | 0,33 | 0,33 |
| Human serum albumin [mg/ml] | 3 | 3 |
| Progesterone [µM] | 0 | 10 |

### Flowchart for performing the CatSper activity test

The flowchart as depicted below is considered as guidance for performing the method of the present invention. It represents the preferred embodiment of the method of the present invention. Number coding: 1 = calcium-containing control solution; 2 = calcium-free test solution in the presence of progesterone (10 µM).

For performing a CatSper-Activity-Test, 450 µl of the calcium-containing control solution (labelled as "1") and the calcium-free test solution (labelled "as 2") are used, respectively (see particular composition above in Table 1). Both solutions are stored in a kit at 4 °C and heated to 37 °C before use.

A sperm (semen) sample is then obtained from a male subject. The initial motility is determined in the native ejaculate with methods established for standard sperm analysis. Samples with less than 10 % motile sperm are excluded from the test. A volume of 50 µl of the native ejaculate is added to the buffer solutions (1 and 2) in each microreaction tube. By low-speed vortex mixing or repeatedly inverting the microreaction tubes, a homogeneous sperm suspension is generated. Both microreaction tubes are then incubated at 37 °C for 15 min. After incubation, the mixing step is repeated and the sperm motility is determined for both conditions by counting a total number of 200 sperm. Condition 1 (= calcium-containing control solution, labelled as "1") is counted prior to condition 2 (= calcium-free test solution, labelled as "2"). Finally, a CatSper-Activity-Index (CAI) is calculated as described elsewhere. Results of the characterization of CATSPER2-deficient human sperm by this CatSper-Activity-Index are depicted in **Fig. 4****.**

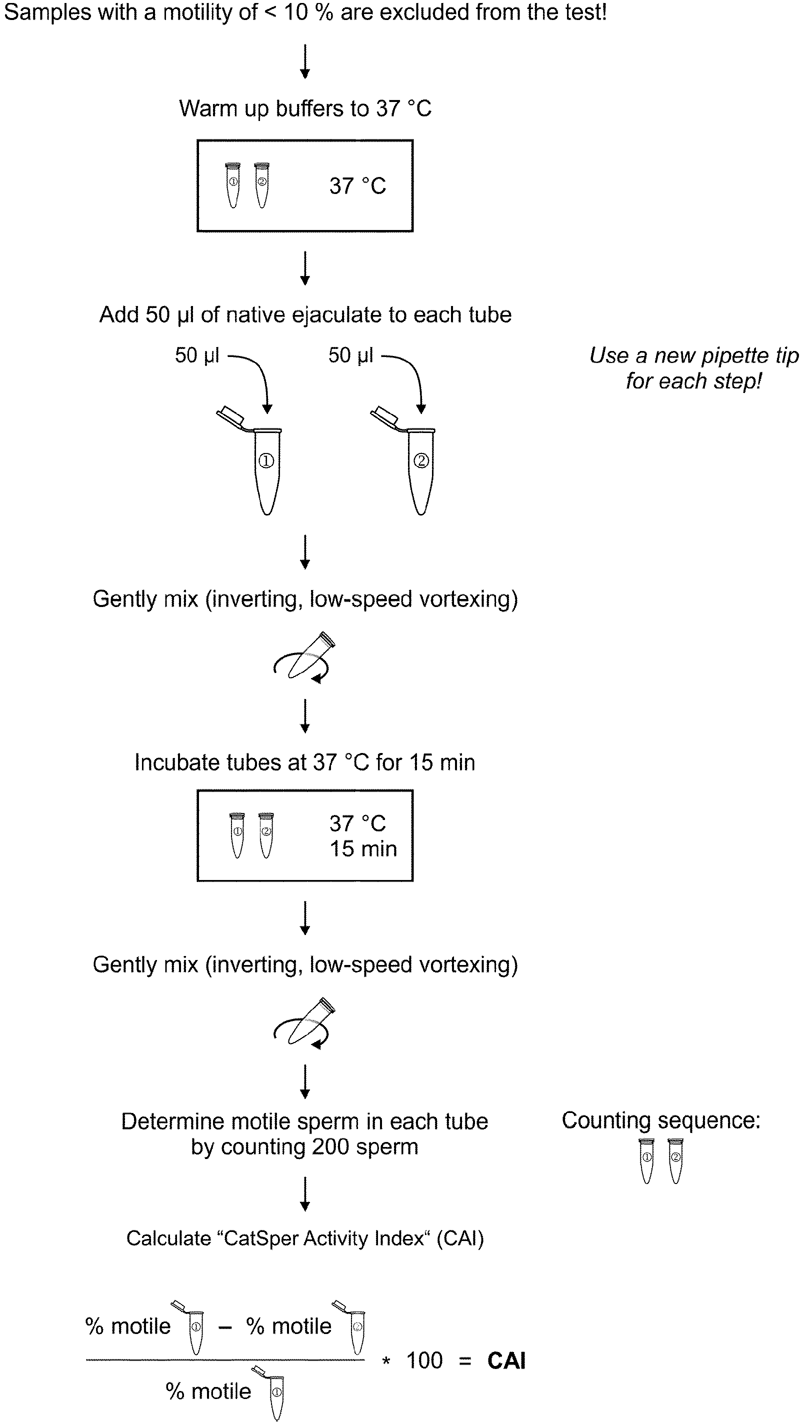

### Verification of CatSper deficiency by electrophysiological recordings and genetic analysis

In order to verify CatSper deficiency, five patients (patients A-E) which we identified as "CatSper deficient" when using the CatSper-Activity-Test (open triangles in Fig. 4) have been additionally analysed by electrophysiological recordings (Fig. 5). For comparison, recordings for sperm from a control subject with an intact CatSper channel are shown (control). Panel A (left) shows monovalent CatSper currents measured in whole-cell derivatives. The current-voltage curves derived from this are shown on the right. Panel B shows the maximum current amplitudes in the inward and outward direction in sperm from CatSper-deficient patients (CATSPER^{-/-}). As a control, current amplitudes of sperm from seven test subjects with an intact CatSper channel are listed (Control). No CatSper currents could be measured in the CatSper-deficient sperm. Electrophysiological recordings were performed as described in Strünker et al., 2011.
In addition, these five patients have been genetically analysed (Fig. 6). So-called array-CGH analyzes (in accordance with e.g., Tüttelmann *et al.* 2011) show that all five patients have a chromosomal deletion that includes the CATSPER2 gene (the CatSper 2 protein is an essential subunit of the CatSper channel complex). In sum, CatSper deficiency determined in accordance with the means and methods of the present invention could be verified by electrophysiological recordings as well as genetic analysis.

## Claims

1. An *in vitro* method for determining or diagnosing infertility of a male subject comprising the steps of:
a) contacting a sperm sample obtained from said subject with a calcium-free test solution,
b) detecting the motility of the sperm in said sample under the test condition of step (a), and
c) determining infertility of the male subject based on the motility detected in step (b).

2. The method according to claim 1, wherein no significant change in the amount of motile sperm under the test condition of step (b) is indicative for infertility of said subject.

3. The method according to claim 1 or 2, wherein a calcium-free test solution comprises less than about 20 nM free calcium, and/or wherein the calcium-free test solution comprises at least one agent reducing the free calcium concentration, wherein the at least one agent reducing the free calcium concentration is preferably a chemical binding Ca²⁺, wherein the chemical binding Ca²⁺ is preferably any one of EGTA, EDTA, BAPTA, HEDTA, NTA, DiBrBAPTA, citric acid, phosphate, or a Ca²⁺-indicator dye, preferably EGTA.

4. The method according to any one of the preceding claims, wherein step (a) further comprises applying at least one activation stimulus of the CatSper channel, wherein the activation stimulus is preferably applied prior to, simultaneously or after applying the calcium-free test solution to the sperm sample.

5. The method according to claim 4, wherein the CatSper activation stimulus is any substance and/or condition increasing the CatSper channel activity, wherein
(a) the substance increasing the CatSper channel activity is preferably a ligand, wherein the ligand is preferably any one of a steroid, prostaglandin, a synthetic or a natural chemical that mimics the action of a steroid or prostaglandin, a serum albumin, a zona pellucida protein, or a combination thereof, wherein the steroid is preferably progesterone, and wherein
(b) the condition increasing the CatSper channel activity preferably comprises increasing the intracellular pH and/or depolarizing the membrane potential.

6. The method according to claim 4 or 5, wherein the sperm sample is contacted for at least about 5 minutes with the test solution of step (a).

7. The method according to any one of claims 2-6, wherein motile sperm is **characterized by** any movement in comparison to motionless sperm.

8. The method according to any one of the preceding claims, wherein the motility of the sperm is determined using a light microscope, and/or wherein determining the amount of motile sperm comprises counting at least about 10 sperm under the test condition of step (b).

9. The method according to any one of the preceding claims, further comprising comparing the motility of the sperm detected in step (b) to the motility of the sperm from said subject detected under a control condition comprising a calcium-containing solution, wherein the comparison of the motility detected under the test condition and the control condition is preferably indicative for infertility of said subject.

10. The method according to claim 9, wherein the comparison of the motility of the sperm under the test condition and the control condition comprises determining a CatSper activity index, comprising the steps of
a) obtaining a percentage value of motile sperm under i) the test condition and ii) the control condition;
b) subtracting the percentage value of motile sperm under the test condition of i) from the percentage value of motile sperm under the control condition of ii), thereby obtaining a subtraction score;
c) determining the ratio of the subtraction score to the percentage value of motile sperm under the control condition of ii); and
d) multiplying the ratio of step c) with the number 100, thereby obtaining the CatSper-Activity-Index,
further preferably comprising comparing the index with a predetermined reference value, wherein the index below the reference value is preferably indicative for infertility of said subject, wherein the reference value is preferably about 20%.

11. The method according to any one of the preceding claims, wherein the male subject is a vertebrate, wherein the vertebrate is preferably a mammal, wherein the mammal is preferably any one of a human, a cattle, a buffalo, a horse, a donkey, a camel, a sheep, a goat, a pig, a dog, a cat, a rat, or a mouse, preferably a human.

12. A kit for use in a method for determining or diagnosing infertility of a male subject comprising
(a) a calcium-free test solution, and
(b) a calcium-containing control solution, and
further comprising a validation solution comprising a CatSper-inhibitor, and
further preferably comprising instructions for use and/or an imprint.

13. The kit for use according to claim 12, wherein the calcium-free test solution comprises at least one agent reducing the free calcium concentration, wherein the at least one agent reducing the free calcium concentration is preferably a chemical binding Ca²⁺, wherein the chemical binding Ca²⁺ is preferably any one of EGTA, EDTA, BAPTA, HEDTA, NTA, DiBrBAPTA, citric acid, phosphate, or a Ca²⁺-indicator dye, preferably EGTA.

14. The kit for use according to claims 12 or 13, wherein the calcium-free test solution and/or the calcium-containing control solution comprises at least one activation stimulus of the CatSper channel, wherein the activation stimulus is preferably any one of a ligand, a weak base, a buffer comprising increased potassium concentration, a potassium- or chloride ion channel inhibitor, or a sodium ionophore, wherein the ligand is preferably any one of a steroid, prostaglandin, a synthetic or a natural chemical that mimics the action of a steroid or prostaglandin, a serum albumin, a zona pellucida protein, or a combination thereof, wherein the steroid is preferably progesterone.

15. Use of a kit comprising
(a) a calcium-free test solution, and
(b) a calcium-containing control solution in an *in vitro* method for determining or diagnosing infertility of a male subject.

## Patentansprüche

1. Ein *in-vitro*-Verfahren zur Bestimmung oder Diagnose der Unfruchtbarkeit eines männlichen Subjekts umfasst die folgenden Schritte:
a) Kontaktieren einer Spermienprobe von besagtem Subjekt mit einer kalziumfreien Testlösung,
b) Nachweis der Beweglichkeit der Spermien in der genannten Probe unter der Testbedingung von Schritt (a), und
c) Feststellung der Unfruchtbarkeit des männlichen Subjekts anhand der im Schritt (b) festgestellten Beweglichkeit.

2. Das Verfahren nach Anspruch 1, bei der keine signifikante Veränderung der Menge beweglicher Spermien unter der Testbedingung von Schritt (b) auf Unfruchtbarkeit des betreffenden Subjekts hinweist.

3. Das Verfahren nach Anspruch 1 oder 2, wobei eine kalziumfreie Testlösung weniger als etwa 20 nM freies Kalzium umfasst und/oder wobei die kalziumfreie Testlösung mindestens ein Mittel umfasst, das die freie Kalziumkonzentration reduziert, wobei das mindestens eine Mittel, das die freie Kalziumkonzentration reduziert, vorzugsweise ein chemisches Bindemittel für Ca²⁺ ist, wobei das chemische Bindemittel für Ca²⁺ vorzugsweise eines von EGTA, EDTA, BAPTA, HEDTA, NTA, DiBrBAPTA, Zitronensäure, Phosphat oder ein Ca²⁺-Indikatorfarbstoff, vorzugsweise EGTA, ist.

4. Das Verfahren nach einem der vorstehenden Ansprüche, wobei Schritt (a) ferner das Anwenden mindestens eines Aktivierungsreizes des CatSper-Kanals umfasst, wobei der Aktivierungsreiz vorzugsweise vor, gleichzeitig oder nach der Zugabe der kalziumfreien Testlösung zu der Spermaprobe erfolgt.

5. Das Verfahren nach Anspruch 4, wobei der CatSper-Aktivierungsreiz eine beliebige Substanz und/oder Bedingung ist, die die CatSper-Kanalaktivität erhöht, wobei
(a) die Substanz, die die CatSper-Kanalaktivität erhöht, vorzugsweise ein Ligand ist, wobei der Ligand vorzugsweise ein Steroid, ein Prostaglandin, eine synthetische oder natürliche Chemikalie, die die Wirkung eines Steroids oder Prostaglandins nachahmt, ein Serumalbumin, ein Zona-Pellucida-Protein oder eine Kombination davon ist, wobei das Steroid vorzugsweise Progesteron ist, und wobei
(b) der Zustand, der die CatSper-Kanalaktivität erhöht, vorzugsweise eine Erhöhung des intrazellulären pH-Werts und/oder eine Depolarisierung des Membranpotentials umfasst.

6. Das Verfahren nach Anspruch 4 oder 5, wobei die Spermaprobe mindestens etwa 5 Minuten lang mit der Testlösung aus Schritt (a) in Kontakt gebracht wird.

7. Das Verfahren nach einem der Ansprüche 2 bis 6, wobei bewegliche Spermien durch jede Bewegung im Vergleich zu unbeweglichen Spermien gekennzeichnet sind.

8. Das Verfahren nach einem der vorstehenden Ansprüche, wobei die Beweglichkeit der Spermien unter Verwendung eines Lichtmikroskops bestimmt wird und/oder wobei die Bestimmung der Menge an beweglichen Spermien das Zählen von mindestens etwa 10 Spermien unter den Testbedingungen von Schritt (b) umfasst.

9. Das Verfahren nach einem der vorstehenden Ansprüche, das ferner das Vergleichen der in Schritt (b) festgestellten Beweglichkeit der Spermien mit der Beweglichkeit der Spermien des Subjekts umfasst, die unter einer Kontrollbedingung mit einer kalziumhaltigen Lösung festgestellt wurde, wobei der Vergleich der unter der Testbedingung und der Kontrollbedingung festgestellten Beweglichkeit vorzugsweise auf die Unfruchtbarkeit des Subjekts hinweist.

10. Das Verfahren nach Anspruch 9, wobei der Vergleich der Motilität der Spermien unter der Testbedingung und der Kontrollbedingung die Bestimmung eines CatSper-Aktivitätsindex umfasst, der die folgenden Schritte umfasst
a) Ermitteln eines prozentualen Werts der beweglichen Spermien unter i) der Testbedingung und ii) der Kontrollbedingung;
b) Subtrahieren des prozentualen Werts der beweglichen Spermien unter der Testbedingung i) vom prozentualen Wert der beweglichen Spermien unter der Kontrollbedingung ii), wodurch ein Subtraktionswert erhalten wird;
c) Bestimmen des Verhältnisses des Subtraktionswerts zum prozentualen Wert der beweglichen Spermien unter der Kontrollbedingung ii); und
d) Multiplizieren des Verhältnisses aus Schritt c) mit der Zahl 100, wodurch der CatSper-Aktivitätsindex erhalten wird,
vorzugsweise ferner umfassend das Vergleichen des Index mit einem vorbestimmten Referenzwert, wobei der Index unterhalb des Referenzwerts vorzugsweise auf eine Unfruchtbarkeit des Subjekts hinweist, wobei der Referenzwert vorzugsweise etwa 20 % beträgt.

11. Das Verfahren nach einem der vorstehenden Ansprüche, wobei das männliche Subjekt ein Wirbeltier ist, wobei das Wirbeltier vorzugsweise ein Säugetier ist, wobei das Säugetier vorzugsweise ein Mensch, ein Rind, ein Büffel, ein Pferd, ein Esel, ein Kamel, ein Schaf, eine Ziege, ein Schwein, ein Hund, eine Katze, eine Ratte oder eine Maus ist, vorzugsweise ein Mensch.

12. Ein Kit zur Verwendung bei einem Verfahren zur Bestimmung oder Diagnose der Unfruchtbarkeit eines männlichen Subjekts, umfassend
(a) eine kalziumfreie Testlösung und
(b) eine kalziumhaltige Kontrolllösung, und
weiterhin umfassend eine Validierungslösung, die einen CatSper-Inhibitor umfasst, und weiterhin vorzugsweise umfassend Gebrauchsanweisungen und/oder einen Aufdruck.

13. Das Kit zur Verwendung gemäß Anspruch 12, wobei die kalziumfreie Testlösung mindestens ein Mittel umfasst, das die freie Kalziumkonzentration reduziert, wobei das mindestens eine Mittel, das die freie Kalziumkonzentration reduziert, vorzugsweise ein chemisches Bindemittel für Ca²⁺ ist, wobei das chemische Bindemittel für Ca²⁺ vorzugsweise eines von EGTA, EDTA, BAPTA, HEDTA, NTA, DiBrBAPTA, Zitronensäure, Phosphat oder ein Ca²⁺-Indikatorfarbstoff, vorzugsweise EGTA, ist.

14. Das Kit zur Verwendung gemäß Anspruch 12 oder 13, wobei die kalziumfreie Testlösung und/oder die kalziumhaltige Kontrolllösung mindestens einen Aktivierungsreiz für den CatSper-Kanal umfasst, wobei der Aktivierungsreiz vorzugsweise einer der folgenden Stoffe ist: ein Ligand, eine schwache Base, ein Puffer mit erhöhter Kaliumkonzentration, ein Kalium- oder Chloridionenkanal-Inhibitor oder ein Natriumionophor, wobei der Ligand vorzugsweise einer der folgenden Stoffe ist: ein Steroid, Prostaglandin, eine synthetische oder natürliche Chemikalie, die die Wirkung eines Steroids oder Prostaglandins nachahmt, ein Serumalbumin, ein Zona-Pellucida-Protein oder eine Kombination davon ist, wobei das Steroid vorzugsweise Progesteron ist.

15. Verwendung eines Kits, umfassend
(a) eine kalziumfreie Testlösung und
(b) eine kalziumhaltige Kontrolllösung in einem In-vitro-Verfahren zur Bestimmung oder Diagnose der Unfruchtbarkeit eines männlichen Subjekts.

## Revendications

1. Procédé *in vitro* pour déterminer ou diagnostiquer l'infertilité d'un sujet masculin, comprenant les étapes suivantes :
a) mettre en contact un échantillon de sperme obtenu à partir dudit sujet avec une solution d'essai sans calcium,
b) détecter la motilité des spermatozoïdes dans ledit échantillon dans les conditions d'essai de l'étape (a), et
c) déterminer l'infertilité du sujet masculin sur la base de la motilité détectée à l'étape (b).

2. Procédé selon la revendication 1, dans lequel aucune variation significative de la quantité de spermatozoïdes mobiles dans les conditions d'essai de l'étape (b) n'est indicative d'une infertilité dudit sujet.

3. Procédé selon la revendication 1 ou 2, dans lequel une solution d'essai sans calcium comprend moins d'environ 20 nM de calcium libre, et/ou dans lequel la solution d'essai sans calcium comprend au moins un agent réduisant la concentration en calcium libre, dans lequel le au moins un agent réduisant la concentration en calcium libre est de préférence un Ca²⁺ à liaison chimique, dans lequel le Ca²⁺ à liaison chimique est de préférence l'un quelconque parmi l'EGTA, l'EDTA, le BAPTA, l'HEDTA, le NTA, le DiBrBAPTA, l'acide citrique, le phosphate ou un colorant indicateur de Ca²⁺, de préférence l'EGTA.

4. Procédé selon l'une quelconque des revendications précédentes, dans lequel l'étape (a) comprend en outre l'application d'au moins un stimulus d'activation du canal CatSper, dans lequel le stimulus d'activation est de préférence appliqué avant, simultanément ou après l'application de la solution d'essai sans calcium à l'échantillon de sperme.

5. Procédé selon la revendication 4, dans lequel le stimulus d'activation CatSper est toute substance et/ou condition augmentant l'activité du canal CatSper, dans lequel
(a) la substance augmentant l'activité du canal CatSper est de préférence un ligand, dans lequel le ligand est de préférence l'un quelconque parmi un stéroïde, une prostaglandine, un produit chimique synthétique ou naturel qui imite l'action d'un stéroïde ou d'une prostaglandine, une albumine sérique, une protéine de la zone pellucide, ou une combinaison de ceux-ci, dans lequel le stéroïde est de préférence la progestérone,
et dans lequel
(b) la condition augmentant l'activité du canal CatSper comprend de préférence l'augmentation du pH intracellulaire et/ou la dépolarisation du potentiel membranaire.

6. Procédé selon la revendication 4 ou 5, dans lequel l'échantillon de sperme est mis en contact pendant au moins environ 5 minutes avec la solution d'essai de l'étape (a).

7. Procédé selon l'une quelconque des revendications 2 à 6, dans lequel le sperme mobile est **caractérisé par** tout mouvement par rapport au sperme immobile.

8. Procédé selon l'une quelconque des revendications précédentes, dans lequel la motilité des spermatozoïdes est déterminée à l'aide d'un microscope optique, et/ou dans lequel la détermination de la quantité de spermatozoïdes mobiles comprend le comptage d'au moins environ 10 spermatozoïdes dans les conditions d'essai de l'étape (b).

9. Procédé selon l'une quelconque des revendications précédentes, comprenant en outre la comparaison de la motilité des spermatozoïdes détectée à l'étape (b) avec la motilité des spermatozoïdes provenant dudit sujet détectée dans des conditions de contrôle comprenant une solution contenant du calcium, dans lequel la comparaison de la motilité détectée dans les conditions d'essai et dans les conditions de contrôle est de préférence indicative de l'infertilité dudit sujet.

10. Procédé selon la revendication 9, dans lequel la comparaison de la motilité des spermatozoïdes dans les conditions d'essai et dans les conditions de contrôle comprend la détermination d'un indice d'activité CatSper, comprenant les étapes suivantes
a) obtenir une valeur en pourcentage des spermatozoïdes mobiles dans i) les conditions d'essai et ii) les conditions de contrôle ;
b) soustraire la valeur en pourcentage des spermatozoïdes mobiles dans la condition d'essai i) de la valeur en pourcentage des spermatozoïdes mobiles dans la condition de contrôle ii), obtenant ainsi un score de soustraction ;
c) déterminer le rapport entre le score de soustraction et la valeur en pourcentage des spermatozoïdes mobiles dans la condition de contrôle ii) ; et
d) multiplier le rapport de l'étape c) par le nombre 100, obtenant ainsi l'indice d'activité CatSper,
comprenant de préférence en outre la comparaison de l'indice avec une valeur de référence prédéterminée, dans lequel l'indice inférieur à la valeur de référence est de préférence indicatif de l'infertilité dudit sujet, dans lequel la valeur de référence est de préférence d'environ 20 %.

11. Procédé selon l'une quelconque des revendications précédentes, dans lequel le sujet mâle est un vertébré, dans lequel le vertébré est de préférence un mammifère, dans lequel le mammifère est de préférence l'un quelconque parmi un être humain, un bovin, un buffle, un cheval, un âne, un chameau, un mouton, une chèvre, un porc, un chien, un chat, un rat ou une souris, de préférence un être humain.

12. Kit destiné à être utilisé dans une méthode de détermination ou de diagnostic de l'infertilité d'un sujet masculin, comprenant
(a) une solution d'essai sans calcium, et
(b) une solution de contrôle contenant du calcium, et
comprenant en outre une solution de validation comprenant un inhibiteur de CatSper, et comprenant de préférence en outre des instructions d'utilisation et/ou une impression.

13. Kit destiné à être utilisé selon la revendication 12, dans lequel la solution d'essai sans calcium comprend au moins un agent réduisant la concentration en calcium libre, dans lequel ledit au moins un agent réduisant la concentration en calcium libre est de préférence un Ca²⁺ à liaison chimique, dans lequel ledit Ca²⁺ à liaison chimique est de préférence l'un quelconque parmi l'EGTA, l'EDTA, le BAPTA, l'HEDTA, le NTA, le DiBrBAPTA, l'acide citrique, le phosphate ou un colorant indicateur de Ca²⁺, de préférence l'EGTA.

14. Kit destiné à être utilisé selon les revendications 12 ou 13, dans lequel la solution d'essai sans calcium et/ou la solution de contrôle contenant du calcium comprend au moins un stimulus d'activation du canal CatSper, dans lequel le stimulus d'activation est de préférence l'un quelconque parmi un ligand, une base faible, un tampon comprenant une concentration accrue en potassium, un inhibiteur de canal potassique ou chlorure, ou un ionophore sodique, dans lequel le ligand est de préférence l'un quelconque parmi un stéroïde, prostaglandine, un produit chimique synthétique ou naturel qui imite l'action d'un stéroïde ou d'une prostaglandine, une albumine sérique, une protéine de la zone pellucide, ou une combinaison de ceux-ci, dans lequel le stéroïde est de préférence la progestérone.

15. Utilisation d'un kit comprenant
(a) une solution d'essai sans calcium, et
(b) une solution de contrôle contenant du calcium dans une méthode *in vitro* pour déterminer ou diagnostiquer l'infertilité d'un sujet masculin.
